# EUROPEAN PATENT SPECIFICATION

(11) **EP 2 096 170 B1**
(45) Date of publication and mention of the grant of the patent: **10.08.2011**
(21) Application number: 09007461.8
(22) Date of filing: 19.09.2006
(51) Int. Cl.: C12N 15/11, A61K 31/712

(54) **Modulation of glucagon receptor expression**
Modulation der Glucagonrezeptorexpression
Modulation d'expression de récepteur de glucagon

(30) Priority: 19.09.2005 US 718684 P
(43) Date of publication of application: 02.09.2009
(62) Divisional of application: 06814974.9
(73) Proprietor: Isis Pharmaceuticals, Inc., Carlsbad, CA 92008 (US)
(72) Inventor: Monia, Brett P., Encinitas, CA 92024 (US); Freier, Susan M., San Diego, CA 92122 (US); Bhanot, Sanjay, Carlsbad, CA 92009 (US)
(74) Representative: Hallybone, Huw George

(56) References cited:
- WO-A-2004/096996
- WO-A-2005/023995
- LIANG Y ET AL: "Reduction in Glucagon Receptor Expression by an Antisense Oligonucleotide Ameliorates Diabetic Syndrome in db/db Mice" DIABETES, NEW YORK, NY, US, vol. 53, February 2004 (2004-02), pages 410-417, XP002995165 ISSN: 0012-1797

### SEQUENCE LISTING

<110> Isis Pharmaceuticals, Inc.
   Monia, Brett P.
   Freier, Susan M.
   Bhanot, Sanjay
<120> MODULATION OF GLUCAGON RECEPTOR EXPRESSION
<130> BIOL0066WO
<150> 60/718,684
   <151> 2005-09-19
<160> 32
<170> FastSEQ for Windows Version 4.0
<210> 1
   <211> 2034
   <212> DNA
   <213> H. sapiens
<400> 1
   ggatctggca gcgccgcgaa gacgagcggt caccggcgcc cgacccgagc gcgcccagag 60
   gacggcgggg agccaagccg acccccgagc agcgccgcgc gggccctgag gctcaaaggg 120
   gcagcttcag gggaggacac cccactggcc aggacgcccc aggctctgct gctctgccac 180
   tcagctgccc tcggaggagc gtacacacac accaggactg cattgcccca gtgtgcagcc 240
   cctgccagat gtgggaggca gctagctgcc cagaggcatg cccccctgcc agccacagcg 300
   acccctgctg ctgttgctgc tgctgctggc ctgccagcca caggtcccct ccgctcaggt 360
   gatggacttc ctgtttgaga agtggaagct ctacggtgac cagtgtcacc acaacctgag 420
   cctgctgccc cctcccacgg agctggtgtg caacagaacc ttcgacaagt attcctgctg 480
   gccggacacc cccgccaata ccacggccaa catctcctgc ccctggtacc tgccttggca 540
   ccacaaagtg caacaccgct tcgtgttcaa gagatgcggg cccgacggtc agtgggtgcg 600
   tggaccccgg gggcagcctt ggcgtgatgc ctcccagtgc cagatggatg gcgaggagat 660
   tgaggtccag aaggaggtgg ccaagatgta cagcagcttc caggtgatgt acacagtggg 720
   ctacagcctg tccctggggg ccctgctcct cgccttggcc atcctggggg gcctcagcaa 780
   gctgcactgc acccgcaatg ccatccacgc gaatctgttt gcgtccttcg tgctgaaagc 840
   cagctccgtg ctggtcattg atgggctgct caggacccgc tacagccaga aaattggcga 900
   cgacctcagt gtcagcacct ggctcagtga tggagcggtg gctggctgcc gtgtggccgc 960
   ggtgttcatg caatatggca tcgtggccaa ctactgctgg ctgctggtgg agggcctgta 1020
   cctgcacaac ctgctgggcc tggccaccct ccccgagagg agcttcttca gcctctacct 1080
   gggcatcggc tggggtgccc ccatgctgtt cgtcgtcccc tgggcagtgg tcaagtgtct 1140
   gttcgagaac gtccagtgct ggaccagcaa tgacaacatg ggcttctggt ggatcctgcg 1200
   gttccccgtc ttcctggcca tcctgatcaa cttcttcatc ttcgtccgca tcgttcagct 1260
   gctcgtggcc aagctgcggg cacggcagat gcaccacaca gactacaagt tccggctggc 1320
   caagtccacg ctgaccctca tccctctgct gggcgtccac gaagtggtct ttgccttcgt 1380
   gacggacgag cacgcccagg gcaccctgcg ctccgccaag ctcttcttcg acctcttcct 1440
   cagctccttc cagggcctgc tggtggctgt cctctactgc ttcctcaaca aggaggtgca 1500
   gtcggagctg cggcggcgtt ggcaccgctg gcgcctgggc aaagtgctat gggaggagcg 1560
   gaacaccagc aaccacaggg cctcatcttc gcccggccac ggccctccca gcaaggagct 1620
   gcagtttggg aggggtggtg gcagccagga ttcatctgcg gagaccccct tggctggtgg 1680
   cctccctaga ttggctgaga gccccttctg aaccctgctg ggaccccagc tagggctgga 1740
   ctctggcacc cagaggcgtc gctggacaac ccagaactgg acgcccagct gaggctgggg 1800
   gcgggggagc caacagcagc ccccacctac cccccacccc cagtgtggct gtctgcgaga 1860
   ttgggcctcc tctccctgca cctgccttgt ccctggtgca gaggtgagca gaggagtcca 1920
   gggcgggagt gggggctgtg ccgtgaactg cgtgccagtg tccccacgta tgtcggcacg 1980
   tcccatgtgc atggaaatgt cctccaacaa taaagagctc aagtggtcac cgtg       2034
<210> 2
   <211> 20
   <212> DNA
   <213> Artificial Sequence
<220>
   <223> Oligomeric compound
<400> 2
   gcactttgtg gtgccaaggc 20
<210> 3
   <211> 1875
   <212> DNA
   <213> R. norvegicus
<400> 3
   gaattcgcgg ccgccgccgg gccccagatc ccagtgcgcg aggagcccag tcctagaccc 60
   agcaacctga ggagaggtgc acacaccccc aaggacccag gcacccaacc tctgccagat 120
   gtgggggggt ggctacccag aggcatgctc ctcacccagc tccactgtcc ctacctgctg 180
   ctgctgctgg tggtgctgtc atgtctgcca aaggcaccct ctgcccaggt aatggacttt 240
   ttgtttgaga agtggaagct ctatagtgac cagtgccacc acaacctaag cctgctgccc 300
   ccacctactg agctggtctg caacagaact ttcgacaagt actcctgctg gcctgacacc 360
   cctcccaaca ccactgccaa catttcctgc ccctggtacc taccttggta ccacaaagtg 420
   cagcaccgcc tagtgttcaa gaggtgtggg cctgatgggc agtgggttcg agggccacgg 480
   gggcagtcat ggcgcgacgc ctcccaatgt cagatggatg atgacgagat cgaggtccag 540
   aagggggtag ccaagatgta tagcagctac caggtgatgt acactgtggg ctacagtctg 600
   tccctggggg ccttgctcct ggcgctggtc atcctgctgg gcctcaggaa gctgcactgc 660
   acccggaact acatccacgg gaacctgttc gcgtccttcg tgctcaaggc tggctctgtg 720
   ctggtcattg attggctgct caagacacgc tatagccaga agattggaga tgacctcagt 780
   gtgagcgtct ggctcagtga tggggcggtg gctggctgca gagtggccac agtgatcatg 840
   cagtacggca tcatagccaa ctactgctgg ttgctggtgg agggtgtgta cctgtacagc 900
   ctgctgagca tcaccacctt ctcggagaag agcttcttct ccctctatct gtgcatcggc 960
   tggggatctc ccctgctgtt tgtcatcccc tgggtggtgg tcaagtgtct gtttgagaat 1020
   gtccagtgct ggaccagcaa tgacaatatg ggattctggt ggatcctgcg tatccctgta 1080
   ctcctggcca tactgatcaa ttttttcatc tttgtccgca tcattcatct tcttgtggcc 1140
   aagctgcgtg cccatcagat gcactatgct gattacaagt tccggctagc caggtccacg 1200
   ctgaccctca ttcctctgct gggagtccac gaagtggtct ttgcctttgt gactgatgag 1260
   catgcccagg gcaccctgcg ctccaccaag ctcttttttg acctgttctt cagctccttt 1320
   cagggtctgc tggtggctgt tctctactgt ttcctcaaca aggaggtgca ggcagagcta 1380
   ctgcggcgtt ggaggcgatg gcaagaaggc aaagctcttc aggaggaaag gatggccagc 1440
   agccatggca gccacatggc cccagcaggg acttgtcatg gtgatccctg tgagaaactt 1500
   cagcttatga gtgcaggcag cagcagtggg actggctgtg agccctctgc gaagacctca 1560
   ttggccagta gtctcccaag gctggctgac agccccacct gaatctccac tggactccag 1620
   ccaagttgga ttcagaaagg gcctcacaag acaacccaga aacagatgcc tggccaaggc 1680
   tgaagaggca aagcagcaag acagcagctt gtactatcca cactccccta acctgtcctg 1740
   gccgggtaca ggccacattg atggagtagg ggctggatat gatggagtag ccatgctatg 1800
   aactatgggt gttcccatga gtgttgccat gttccatgca cacagatatg accttcagta 1860
   aagagctccc gtagg                                                  1875
<210> 4
   <211> 20
   <212> DNA
   <213> Artificial Sequence
<220>
   <223> Oligomeric compound
<400> 4
   gcactttgtg gtaccaaggt 20
<210> 5
   <211> 2378
   <212> DNA
   <213> H. sapiens
<400> 5
   cctctagagt caacatgaca ggcatcgaat ggctcctgtt tctctggcag agttgggggc 60
   agagccaggc ttggccacgc tgggctctaa ggggctgtca ttttgcccag ggagctcctg 120
   gctgggtggt cctcccccca gggtgagcac gcgtcccccc cacccccact tcgaggcgcc 180
   caggcaggga acagctcatt ggccagtgtc cttcctcctt gtccccgcct gcatctccac 240
   catccaccct gctccagctg ccccttgtcc ctctccccgt cccctgccca gagccccagg 300
   tctcccctgc acccctgagc ctgcccacct agcagtgccc ctcgtccagg gcccctctgg 360
   gttgggggtg cacacagcgg ggagaggcgg ctcctgctgc tcctcaccca gcccggctca 420
   gtggccggag ccgcccagga cagtggcagt agatggggct gtttgatcag gatcagggaa 480
   gataaggccc cttgcgtgac cccagagctg gggacgccaa aactgcccct cttccccyac 540
   ccgcctgccg ctgtctctgc cagggagagg cccctactct gtgggtcctt cgccccagca 600
   ccaagcctgc atggctgctc acctggctca ggaactgggg atcagcgaca cacgggtcct 660
   gcctcccatc ggcccctaca tgagcccagg gtccaagggc tgaggttggg agctctttag 720
   cagtctgtga cgcaggtgcc tgtccctgtc attcagctgt cacactgctt ggggcatctc 780
   aggccccgtt agcggggcag ccctgggtgg agctggcccc acgcgggctc acccagccgc 840
   tacctggagg aggctaaaat ccaggctgtc ccgtggcagc cagcagtcca ggcctgcccg 900
   gaaaccctct gctccagctg cagccttcgc ccatctcctt gcccctctcc ctggcttccc 960
   cctggcactg ccttccagct ggctggccct ccatctgccc agccatccat ccacacctct 1020
   tattccattt gagggtgccc caaagaagag cccgtaacag cccgggggct catagccagc 1080
   cactcgcggg accccgcaca tgcacgtgga cccacaggaa gaccctccct gcttctccca 1140
   cagaattcag ttggtgcaga aactgggctc tgtagcaacg aaaggccgat ttgtgtagct 1200
   gttgccaccc cgaactccca gctcagatgc tggctgtggc atggggacca ggggctgtga 1260
   ctcccacagc cctggcaggc accacggggg atgtcctccc caccctgtgc ccccacccta 1320
   ggccagctcc tcctccaagt cgacgcccgc agtgctaacc tcaaaggact gtgcagccag 1380
   cctgtggcgt cccatgggat ccaggaagcc caaccgagcc ttgcacggca cccacgaggc 1440
   acctaggcac cccggtgctg ggcagggggc acacatgtga cacagacccc tgagtgtggg 1500
   ccccacacac ttggcctggc acagctgcaa gccagcccag ccactttgct cgctgtggca 1560
   ctggggccaa gtgatggaag gtccaggcat cgccaccctc acgcttggca cattggctca 1620
   ggtcagcctg gcaagccagc tttcccaggg gctaagaata ggtgaggagg atggtgagga 1680
   agcacgccgg ggggctgtca actgagggag gaggtcacca tctggggagg ctggtcgccc 1740
   caagagcatt gggtcacctg caggaaggtg gctgccacca gcaatgagac gaggggctct 1800
   gcgaccctca gagctgccag ccagccagcc ctgggtggca agagtgactc ctcctggggt 1860
   ctcctccctc ctatcgccct cttttttttt tttttttttt ttgagacgga gtctcgctct 1920
   gcacagctga ctgcaatgct gatctcgctc actgcaaggt ctgccccggg ttcacgccat 1980
   tctcactgcc acaagctccc gagtagctgg actacagacg cccgccacca cgcctggcta 2040
   attttttgta tttttagtta gagacggggt ttcactgtgt agcggatggt ctcgatctcc 2100
   agacctccgt tgatccaccc ccctcggcct cccaagttct gggaaacagg cgtgagcgcc 2160
   gcgcccggcc cccagctccc tctttatccc taggaccctg aggctcagag gggcagcttc 2220
   aggggaggac accccactgg ccagacgccc caggctctgc tgctctgcca ctcagctgcc 2280
   ctcggaggag cgtacacaca caccaggact gcattgcccc agctgtgcag cccctgccag 2340
   atgtgggagg cagctagctg cccagaggca tgcccccc                         2378
<210> 6
   <211> 735
   <212> DNA
   <213> H. sapiens
<220>
   <221> mise_feature
   <222> 42, 346
   <223> n = A, T, C or G
<400> 6
   cagccacagc gacccctgct gctgttgctg ttgctgctgg cntgcagggt cccctccgct 60
   caggtgatgg acttcctgtt tgagaagtgg aagctctacg gtgaccagtg tcaccacaac 120
   ctgagcctgc tgccccctcc cacgggtgag ccccccaccc agagcctttc agcctgtgcc 180
   tggcctcagc acttcctgag ttctcttcat gggaaggttc ctgggtgctt atgcagcctt 240
   tgaggacccc gccaaggggc cctgtcattc ctcaggcccc caccaccgtg ggcagagctg 300
   gtgtgcaaca gaaccttcga caagtattcc tgctggccgg acaccnccgc caataccacg 360
   gccaacatct cctgcccctg gtacctgcct tggcaccaca aagtgcaaca ccgcttcgtg 420
   ttcaagagat gcgggcccga cggtcagtgg gtgcgtggac cccgggggca gccttggcgt 480
   gatgcctccc agtgccagat ggatggcgag gagattgagg tccaggtcag tgggcggcag 540
   gcaggcgcgg tggggctgga tgggaacggg catgggggcc cctgcctggc cctcacaggc 600
   cactgtaact cgcagaagga ggtggccaag atgtacagca gcttccaggt gatgtacaca 660
   gtgggctaca gcctgtccct gggggcgctg ctcctcgcct tggccatcct ggggggcctc 720
   aggtaggatt ccgcc                                                 735
<210> 7
   <211> 11001
   <212> DNA
   <213> H. sapiens
<220>
   <221> misc_feature
   <222> 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339
   <223> n = A, T, C or G
<220>
   <221> misc_feature
   <222> 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386
   <223> n = A,T,C or G
<220>
   <221> misc_feature
   <222> 387, 388, 389, 390, 391, 392, 476, 480, 487, 535, 546, 553, 574, 596, 615, 635, 639, 780, 786, 1568, 1645, 2097, 2555, 2559, 2564, 2653, 2752, 2788, 2797, 2880, 2883, 2886, 2923, 3135, 3178, 3209, 3468, 3750, 3859, 3915, 4023, 4226
   <223> n = A,T,C or G
<220>
   <221> misc_feature
   <222> 4235, 4242, 4352, 4387, 4390, 4418, 4619, 4717, 4883, 5060, 5106, 5148, 5587, 6235, 6417, 6828, 6866, 6880, 6917, 6934, 6976, 6987, 6989, 6992, 6993, 6994, 6997, 6998, 6999, 7000, 7001, 7002, 7003, 7004, 7005, 7006, 7007, 7008, 7010
   <223> n = A,T,C or G
<220>
   <221> misc_feature
   <222> 7011, 7012, 7014, 7015, 7016, 7017, 7018, 7019, 7020, 7021, 7029, 7047, 7053, 7056, 7058, 7070, 7089, 7092, 7099, 7105, 7110, 7111, 7141, 7234, 7235, 7236, 7238, 7239, 7241, 7242, 7243, 7245, 7246, 7247, 7249, 7250, 7251, 7252, 7253
   <223> n = A,T,C or G
<220>
   <221> misc_feature
   <222> 7254, 7255, 7256, 7257, 7258, 7259, 7260, 7262, 7290, 7311, 7320, 7462, 7467, 7477, 7479, 7482, 7485, 7487, 7504, 7937, 8081, 8106, 8233, 8353, 8578, 8628, 8646, 8670, 8673, 8675, 9059, 9397, 9443, 9448, 9773, 9913, 9923, 9925, 9927
   <223> n = A,T,C or G
<400> 7
<210> 8
   <211> 20
   <212> DNA
   <213> Artificial Sequence
<220>
   <223> Oligomeric compound
<400> 8
   cgtgtgtctg tgctagtccc 20
<210> 9
   <211> 20
   <212> DNA
   <213> Artificial Sequence
<220>
   <223> Oligomeric compound
<400> 9
   ggcaacgtga acaggtccaa 20
<210> 10
   <211> 18
   <212> DNA
   <213> Artificial Sequence
<220>
   <223> Oligomeric compound
<400> 10
   gcccattgct ggacatgc 18
<210> 11
   <211> 20
   <212> DNA
   <213> Artificial Sequence
<220>
   <223> Oligomeric compound
<400> 11
   agcccattgc tggacatgca 20
<210> 12
   <211> 20
   <212> DNA
   <213> Artificial Sequence
<220>
   <223> Oligomeric compound
<400> 12
   ttgtcccagt cccaggcctc 20
<210> 13
   <211> 20
   <212> DNA
   <213> Artificial Sequence
<220>
   <223> Oligomeric compound
<400> 13
   ctttccgttg gacccctggg 20
<210> 14
   <211> 20
   <212> DNA
   <213> Artificial Sequence
<220>
   <223> Oligomeric compound
<400> 14
   gtgcgcgcga gcccgaaatc 20
<210> 15
   <211> 20
   <212> DNA
   <213> Artificial Sequence
<220>
   <223> Oligomeric compound
<400> 15
   atccaagtgc tactgtagta 20
<210> 16
   <211> 20
   <212> DNA
   <213> Artificial Sequence
<220>
   <223> Oligomeric compound
<220>
   <221> misc_feature
   <222> 1, 2, 3, 4, 5, 6, 7, 8, 9, 10, 11, 12, 13, 14, 15, 16, 17, 18, 19, 20
   <223> n = A,T,C or G
<400> 16
   nnnnnnnnnn nnnnnnnnnn 20
<210> 17
   <211> 20
   <212> DNA
   <213> Artificial Sequence
<220>
   <223> Oligomeric compound
<400> 17
   gccctccatg ctggcacagg 20
<210> 18
   <211> 20
   <212> DNA
   <213> Artificial Sequence
<220>
   <223> Oligomeric compound
<400> 18
   agcaaaagat caatccgtta 20
<210> 19
   <211> 20
   <212> DNA
   <213> Artificial Sequence
<220>
   <223> Oligomeric compound
<400> 19
   tacagaaggc tgggccttga 20
<210> 20
   <211> 20
   <212> DNA
   <213> Artificial Sequence
<220>
   <223> Oligomeric compound
<400> 20
   atgcattctg cccccaagga 20
<210> 21
   <211> 17
   <212> DNA
   <213> Artificial Sequence
<220>
   <223> PCR primer
<400> 21
   tgcggttccc cgtcttc 17
<210> 22
   <211> 24
   <212> DNA
   <213> Artificial Sequence
<220>
   <223> PCR primer
<400> 22
   cttgtagtct gtgtggtgca tctg 24
<210> 23
   <211> 17
   <212> DNA
   <213> Artificial Sequence
<220>
   <223> PCR probe
<400> 23
   catcttcgtc cgcatcg 17
<210> 24
   <211> 21
   <212> DNA
   <213> Artificial Sequence
<220>
   <223> PCR primer
<400> 24
   cagtgccacc acaacctaag c 21
<210> 25
   <211> 25
   <212> DNA
   <213> Artificial Sequence
<220>
   <223> PCR primer
<400> 25
   agtacttgtc gaaagttctg ttgca 25
<210> 26
   <211> 23
   <212> DNA
   <213> Artificial Sequence
<220>
   <223> PCR probe
<400> 26
   tgctgccccc acctactgag ctg 23
<210> 27
   <211> 16
   <212> DNA
   <213> Artificial Sequence
<220>
   <223> PCR primer
<400> 27
   actgcacccg caacgc 16
<210> 28
<211> 18
   <212> DNA
   <213> Artificial Sequence
<220>
   <223> PCR primer
<400> 28
   cacggagctg gccttcag 18
<210> 29
   <211> 26
   <212> DNA
   <213> Artificial Sequence
<220>
   <223> PCR probe
<400> 29
   atccacgcga acctgtttgt gtcctt 26
<210> 30
   <211> 24
   <212> DNA
   <213> Artificial Sequence
<220>
   <223> PCR primer
<400> 30
   gaaccttcga caagtattcc tgct 24
<210> 31
   <211> 18
   <212> DNA
   <213> Artificial Sequence
<220>
   <223> PCR primer
<400> 31
   gggcaggaga tgttggcc 18
<210> 32
   <211> 22
   <212> DNA
   <213> Artificial Sequence
<220>
   <223> PCR probe
<400> 32
   ccagacaccc ccgccaataa ca 22

## Description

### SEQUENCE LISTING

A compnter-readable form of the sequence listing, on diskette, containing the file named BIOL0066WOSEQ.txt, which is 29,184 bytes (measured in MS-DOS) and was created on September 19,2006.

### FIELD OF THE INVENTION

Disclosed herein are compounds, compositions and methods for modulating the expression of glucagon receptor in a cell, tissue or animal.

### BACKGROUND OF THE INVENTION

The maintenance of normal glycemia is a carefully regulated metabolic event. Glucagon, the 29-amino acid peptide responsible for maintaining blood glucose levels, increases glucose release from the liver by activating hepatic glycogenolysis and gluconeogenesis, and also stimulates lipolysis in adipose tissue. In the fed state, when exogenous glucose is consumed leading to high blood glucose levels, insulin reverses the glucagon-mediated enhancement of glycogenolysis and gluconeogenesis. In patients with diabetes, insulin is either not available or not fully effective. While treatment for diabetes has traditionally focused on increasing insulin levels, antagonism of glucagon function has been considered as an alternative therapy. As glucagon exerts its physiological effects by signaling through the glucagon receptor (also known as GCGR or GR), the glucagon receptor has been proposed as a potential therapeutic target for diabetes (Madsen et al., Curr Pharm. Des., 1999, 5, 683-691).

Glucagon receptor belongs to the superfamily of G-protein-ooupled receptors having seven transmembrane domains. It is also a member of the smaller sub-family of homologous receptors which bind peptides that are structurally similar to glucagon. The gene encoding human glucagon receptor was cloned in 1994 and analysis of the genomic sequence revealed multiple introns and an 82% identity to the rat glucagon receptor gene (Lok et al., Gene, 1994, 140, 203-209.; MacNeil et al., Biochem. Biophys. Res. Commun., 1994,198, 328-334). Cloning of the rat glucagon receptor gene also led to the description of multiple alternative splice variants (Maget et al., FEBS Lett, 1994, 351, 271-275). Disclosed in US Patent 5,776,725 is an isolated nucleic acid sequence encoding a human or rat glucagon receptor (Kindsvogel et al.,**1998**). The human glucagon receptor gene is localized to chromosome 17q25 (Menzel et aL, Genomics. 1994, 20, 321-328). A missense mutation of Gly to Ser at codon 40 in the glucagon receptor gene leads to a 3-fold lower affinity for glucagon (Fujisawa et al., Diabetologia 1995, 38, 983-985) and this mutation has been linked to several disease states, including non-insulin-dependent diabetes mellitus (Fujisawa et al., Diabetologia. 1995, 38, 983-985), hypertension (Chambers and Morris, Nat Genet., 1996, 12, 122), and central adiposity (Siani et al., Obes. Res., 2001, 9, 722-726). Targeted disruption of the glucagon receptor gene in mice has shown that, despite a total absence of glucagon receptors and elevated plasma glucagon levels, the mice maintain near-normal glycemia and lipidemia (Parker et al, Biochem. Biophys. Res. Commun., 2002, 290, 839-843).

### SUMMARY OF THE INVENTION

The invention provides an antisense oligonucleotide 13 to 26 nucleobases in length targeted to a nucleic acid molecule encoding human GCGR, wherein the oligonucleotide comprises a first region, a second region, and a third region, wherein said first region comprises at least 11 deoxynucleotides, wherein said second and third regions comprise 1 to 4 2'-O-(2-methoxyethyl) nucleotides, said second and third regions flanking the first region on the 5' and 3' ends of said first region, wherein said oligonucleotide has at least 85% sequence complementarity to a target region within the nucleic acid molecule encoding human GCGR, and wherein the oligonucleotide specifically hybridizes to and reduces expression of human GCGR.

The invention also provides a pharmaceutical composition comprising the antisense oligonucleotide of the invention and optionally a pharmaceutically acceptable carrier, diluent, enhancer or excipient

The invention also provides the pharmaceutical composition of the invention, for use in reducing the expression of GCGR in tissues or cells.

The invention also provides the use of the pharmaceutical composition of the invention in the manufacture of a medicament for reducing the expression of GCGR in tissues or cells.

The invention also provides the pharmaceutical composition of the invention, for use in:
(i) decreasing blood glucose levels in an animal;
(ii) increasing GLP-1 levels in an animal;
(iii) improving insulin sensitivity in an animal;
(iv) decreasing blood triglycerides in an animal;
(v) decreasing blood cholesterol levels in an animal;
(vi) preventing or delaying the onset of elevated blood glucose levels in an animal; or
(vii) preserving beta-cell function in an animal.

The invention also provides the use of the pharmaceutical composition of the invention in the manufacture of a medicament for:
(i) decreasing blood glucose levels in an animal;
(ii) increasing GLP-1 levels in an animal;
(iii) improving insulin sensitivity in an animal;
(iv) decreasing blood triglycerides in an animal;
(v) decreasing blood cholesterol levels in an animal;
(vi) preventing or delaying the onset of elevated blood glucose levels in an animal; or
(vii) preserving beta-cell function in an animal.

The invention also provides the pharmaceutical composition of the invention, for use in treating an animal having a disease or condition associated with glucagon activity via GCGR.

The invention also provides the use of the pharmaceutical composition of the invention in the manufacture of a medicament for treating an animal having a disease or condition associated with glucagon activity via GCGR.

### SUMMARY OF THE DISCLOSURE

The present disclosure is directed to oligomeric compounds targeted to and hybridizable with a nucleic acid molecule encoding GCGR which modulate the expression of GCGR and possess improved pharmacokinetics as compared to oligonucleotides targeted to GCGR comprising a 10-deoxynucleotide gap region flanked on it's 5' and 3' ends with five 2'-O-(2-methoxyethyl) nucleotides. Disclosed herein are oligonucleotides referred to as "gapmers", comprising a deoxynucleotide region or "gap" flanked on each of its 5' and 3' ends with "wings" comprised of one to four 2'-O-(2-methoxoethyl) nucleotides. The deoxynucleotide regions of the oligonucleotides of the disclosure comprised of greater than ten deoxynucleotides, thus the gapmers of the present invention are "gap-widened" as compared to chimeric compounds comprising a ten deoxynucleotide gap region, such as are exemplified in US Publication 2005-0014713. The kidney concentrations of the gap-widened oligonucleotides targeting GCGR have been found to be decreased with respect to those of oligonucleotides having the same sequence but comprising a ten deoxynucleotide region flanked on both the 5' and 3' ends with five 2'-O-(2 methoxyethyl) nucleotides while maintaining the oligonucleotides' good to excellent potency in the liver. Thus, embodiments described herein include gap-widened oligonucleotides targeting GCGR wherein kidney concentrations of said oligonucleotide are decreased with respect to an oligonucleotide having the same sequence but comprising a ten deoxynucleotide region flanked on both the 5' and 3' ends with five 2'-O-(2-methoxyethyl) nucleotides. Another embodiment of the disclosure includes gap-widened oligonucleotides targeting GCGR wherein kidney concentrations of said oligonucleotide are comparable to or decreased with respect to that of an oligonucleotide having the same sequence but comprising a ten deoxynucleotide region flanked on both the 5' and 3' ends with five 2'-O-(2-methoxyethyl) nucleotides while maintaining or improving potency in target tissues such as liver.

In some embodiments, as compared to oligonucleotides having the same sequence but comprising a ten deoxynucleotide region flanked on both the 5' and 3' ends with five 2'-O-(2-methoxyethyl) nucleotides, gap-widened oligonucleotides have comparable or improved potency without enhanced accumulation of oligonucleotide in the liver. Thus, embodiments of the disclosure include gap-widened oligonucleotides targeting GCGR wherein potency is comparable to or better than that of an oligonucleotide having the same sequence but comprising a ten deoxynucleotide region flanked on both the 5' and 3' ends with five 2'-O-(2-methoxyethyl) nucleotides without enhanced accumulation of oligonucleotide in target tissues.

Further described are methods of modulating the expression of GCGR in cells, tissues or animals comprising contacting said cells, tissues or animals with one or more of the compounds or compositions of the disclosure. For example, in one embodiment, the compounds or compositions of the disclosure can be used to reduce the expression of GCGR in cells, tissues or animals. The disclosure includes a pharmaceutical composition comprising an antisense oligonucleotide of the invention and optionally a pharmaceutically acceptable carrier, diluent, excipient, or enhancer.

In one embodiment, the disclosure describes methods of lowering blood glucose using the oligomeric compounds delineated herein. In another embodiment, the disclosure describes methods of increasing GLP-1 levels using the oligomeric compounds delineated herein.

In other embodiments, the disclosure is directed to methods of ameliorating or lessening the severity of a condition in an animal comprising contacting said animal with an effective amount of an oligomeric compound or a pharmaceutical composition described herein In other embodiments, the disclosure is directed to methods of ameliorating or lessening the severity of a condition in an animal comprising contacting said animal with an effective amount of an oligomeric compound or a pharmaceutical composition described herein so that expression of GCGR is reduced and measurement of one or more physical indicator of said condition indicates a lessening of the seventy of said condition. In some embodiments, the disease or condition is a metabolic disease or condition. In some embodiments, the conditions include, but are not limited to, diabetes, obesity, insulin resistance, and insulin deficiency. In some embodiments, the diabetes is type 2 diabetes. In another embodiment, the condition is metabolic syndrome. In one embodiment, the obesity is diet-induced. Also disclosed are methods of preventing or delaying the onset of elevated blood glucose levels in an animal comprising administering to said animal a compound or pharmaceutical composition Also disclosed is a method of preserving beta-cell function.

The instant application is also related to U.S. Application No. 11/231,243 and PCT Application No. PCT/US2005/033837 .

### DETAILED DESCRIPTION:

### Overview

Disclosed herein are oligomeric compounds, including antisense oligonucleotides and other antisense compounds for use in modulating the expression of nucleic acid molecules encoding GCGR. This is accomplished by providing oligomeric compounds which hybridize with one or more target nucleic acid molecules encoding GCGR.

In accordance with the present invention are compositions and methods for modulating the expression of GCGR (also known as glucagon receptor or GR). Listed in Table 1 are GENBANK® accession numbers of sequences which may be used to design oligomeric compounds targeted to GCGR. Oligomeric compounds of the invention include oligomeric compounds which hybridize with one or more human target nucleic acid molecules shown in Table 1, as well as Oligomeric compounds which hybridize to other nucleic acid molecules encoding human GCGR.

The oligomeric compounds may target any region, segment, or site of nucleic acid molecules which encode GCGR. Suitable target regions, segments, and sites include, but are not limited to, the 5'UTR, the start codon, the stop codon, the coding region, the 3'UTR, the 5'cap region, introns, exons, intron-exon junctions, exon-intron junctions, and exon-exon junctions.

**Table 1**

| **Gene Targets** | | |
|---|---|---|
| **Species** | **GENBANK® Accession Number or Description** | **SEQ ID NO** |
| Human | NM 000160.1 | 1 |
| Rat | M96674.1 | 3 |
| Human | AJ45489.1 | 5 |
| Human | The compliment of AI261290.1 | 6 |
| Human | Nucleotides 57000 to 68000 of NT 079568.1 | 7 |

The locations on the target nucleic acid to which active oligomeric compounds hybridize are herein below referred to as "validated target segments." As used herein the term "validated target segment" is defined as at least an 8-nucleobase portion of a target region to which an active oligomeric compound is targeted. While not wishing to be bound by theory, it is presently believed that these target segments represent portions of the target nucleic acid which are accessible for hybridization.

The present invention includes oligomeric compounds which are chimeric compounds. An example of a chimeric compound is a gapmer having a 2'-deoxynucleotide region or "gap" flanked by non-deoxynucleotide regions or "wings". While not wishing to be bound by theory, the gap of the gapmer presents a substrate recognizable by RNase H when bound to the RNA target whereas the wings are not an optimal substrate but can confer other properties such as contributing to duplex stability or advantageous pharmacokinetic effects. Each wing can be one or more non-deoxy oligonucleotide monomers. In one embodiment, the gapmer is comprised of a sixteen 2'-deoxynucleotide region flanked on each of the 5' and 3' ends by wings of two 2'-O-(2-methoxyethyl) nucleotides. This is referred to as a 2-16-2 gapmer. Thus, the "motif" of this chimeric oligomeric compound or gapmer is 2-16-2. In another embodiment, all of the internucleoside linkages are phosphorothioate linkages. In another embodiment the cytosines of the gapmer are 5-methylcytosines.

Embodiments of the present invention include oligomeric compounds comprising sequences of 13 to 26 nucleotides in length and comprising a deoxy nucleotide region greater than 10 nucleobases in length flanked on each of the 5' and 3' ends with at least one 2'-O-(2-methoxyethyl) nucleotide. Preferred "gap-widened" oligonucleotides comprise 11,12,13,14, 15, 16,17, or 18 deoxynucleotides in the gap portion of the oligonucleotide. Also preferred are antisense oligonucleotides 20 nucleobases in length. Preferred 5' and 3' flanking regions comprise 1, 2, 3, or 4 2'-O-(2-methoxyethyl) nucleotides. Preferred gap-widened oligonucleotides have motifs including 1-18-1, 1-17-2, 2-17-1, 2-16-2, 3-14-3, and 4-12-4.

In some embodiments, the oligomeric compounds reduce the expression of GCGR wherein the expression of GCGR is reduced by at least 10%, by at least 20%, by at least 30%, by at least 40%, by at least 50%, by at least 60%, by at least 70%, by at least 80%, by at least 90%, or by 100%.

Oligonucleotides of the present invention preferably include those wherein kidney concentrations of said oligonucleotide are decreased with respect to an oligonucleotide having the same sequence but comprising a ten deoxynucleotide region flanked on both the 5' and 3' ends with five 2'-O-(2-methoxyethyl) nucleotides. Oligonucleotides of the present invention include those wherein kidney concentrations of said oligonucleotide are comparable to or decreased with respect to those of an oligonucleotide having the same sequence but comprising a ten deoxynucleotide region flanked on both the 5' and 3' ends with five 2'-O-(2 methoxyethyl) nucleotides. Oligonucleotides of the present invention include those wherein potency with regard to target reduction in the liver or a therapeutic effect is comparable to or better than that of an oligonucleotide having the same sequence but comprising a ten deoxynucleotide region flanked on both the 5' and 3' ends with five 2'-O-(2-methoxyethyl) nucleotides without enhanced accumulation of oligonucleotide in tissues.

The oligonucleotides of the invention specifically hybridize to 11, GCGR and reduce expression of human GCGR. In some embodiments, the "gap" region comprises 11, 12, 13, 14, 15, 16, 17, or 18 nucleobases. In some embodiments, the antisense oligonucleotides are 20 nucleobases in length.

The oligomeric compounds disclosed herein can comprise about 8 to about 80 nucleobases (i.e. from about 8 to about 80 linked nucleosides), preferably between about 13 to about 26 nucleobases. One of ordinary skill in the art will appreciate that the preferred oligomeric compounds contemplated include compounds that are 13,14, 15,16,17, 18,19, 20, 21, 22, 23, 24, 25 or 26 nucleobases in length.

Compounds of the invention include oligonucleotide sequences that comprise at least the 8 consecutive nucleobases from the 5'-terminus of one of the illustrative antisense compounds (the remaining nucleobases being a consecutive stretch of the same oligonucleotide beginning immediately upstream of the 5'-terminus of the antisense compound which is specifically hybridizable to the target nucleic acid and continuing until the oligonucleotide comprises about 13 to about 26 nucleobases). Other compounds are represented by oligonucleotide sequences that comprise at least the 8 consecutive nucleobases from the 3'-terminus of one of the illustrative antisense compounds (the remaining nucleobases being a consecutive stretch of the same oligonucleotide beginning immediately downstream of the 3-terminus of the antisense compound which is specifically hybridizable to the target nucleic acid and continuing until the oligonucleotide comprises about 13 to about 26 nucleobases). It is also understood that compounds may be represented by oligonucleotide sequences that comprise at least 8 consecutive nucleobases from an internal portion of the sequence of an illustrative compound, and may extend in either or both directions until the oligonucleotide contains about 13 to about 26 nucleobases.

The present invention provides antisense oligonucleotides comprising the nucleobase sequence of SEQ ID NO: 2 . In preferred embodiments, the oligonucleotides of the invention comprise at least an 8-nucleobase portion of the nucleobase sequence of SEQ ID NO: 2.

In a preferred embodiment, the present invention provides antisense oligonucleotides 20 nucleobases in length targeted to a nucleic acid molecule encoding human GCGR and comprising at least an 8-nucleobase portion of SEQ ID NO: 2 wherein the oligonucleotide comprises a deoxynucleotide region 12,13, 15, 16, 17, or 18 nucleobases in length which is flanked on its 5' and 3' ends with 1 to 4 2'-O-(2-methoxyethyl) nucleotides and wherein the oligonucleotide specifically hybridizes to and reduces expression of human GCGR.

In one embodiment, the flanking regions are symmetrical (having the same number of nucleotides in the 5' flanking region as in the 3' flanking region). In another embodiment, the flanking regions are non-symmetrical (having a different number of nucleotides in the 5' flanking region compared to the 3' flanking region).

In other embodiments, the present invention includes antisense oligonucleotides having the nucleobase sequence of SEQ ID NO: 2, wherein the antisense oligonucleotide is characterized by a 12-deoxynncleotide region flanked on its 5' and 3' ends with four 2'-O-(2-methoxyethyl) nucleotides, a 16-deoxynucleotide region flanked on its 5' and 3' ends with two 2'-O-(2 methoxyethyl) nucleotides, a 17-deoxynucleotide region flanked on its 5' and 3' ends with one or two 2'-O-(2-methoxyethyl) nucleotides, or an 18-deoxynucleotide region flanked on its 5' and 3' ends with one 2'-O-(2-methoxyethyl) nucleotides.

Antisense oligonucleotides of the invention may contain at least one modified internucleoside linkage. Modified internucleoside linkages include phosphorothioate linkages. In one embodiment, all internucleoside linkages in an antisense oligonucleotide are phosphorothioate linkages. The antisense oligonucleotides of the invention may also contain at least one modified nucleobase. In one embodiment, at least one cytosine is a 5-methylcytosine. In another embodiment, all cytosines are 5-methylcytosines.

An embodiment of the present invention is an antisense oligonucleotide, 20 nucleobases in length, having the sequence of SEQ ID NO: 2, characterized by a 16-deoxynucleotide region flanked on its 5' and 3' ends with two 2'-O-(2-methoxyethyl) nucleotides wherein each linkage is a phosphorothioate linkage and each cytosine is a 5-methylcytosine.

In a particular embodiment, the antisense oligonucleotides have the nucleobase sequence of SEQ ID: 2, wherein the antisense oligonucleotide has a 12-deoxynucleotide region flanked on its 5' and 3' ends with four 2'-O(2-methoxyethyl) nucleotides. In a further embodiment, the antisense oligonucleotide specifically hybridizes to and reduces expression of GCGR. In a further embodiment, at least one internucleoside linkage is a phosphorothioate linkage. In a further embodiment, at least one cytosine is a 5-methylcytosine.

In a particular embodiments, the antisense oligonucleotide has the nucleobase sequence of SEQ ID: 2, wherein the antisense oligonucleotide has a 14-deoxynucleotide region flanked on its 5' and 3' ends with three 2'-O(2-methoxyethyl) nucleotides. In a further embodiment, the antisense oligonucleotide specifically hybridizes to and reduces expression of GCGR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 2, wherein the antisense oligonucleotide has a 16-deoxynucleotide region flanked on its 5' and 3' ends with two 2'-O(2-methoxyethyl) nucleotides. In a further embodiment the antisense oligonucleotide specifically hybridizes to and reduces expression of GCGR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 2, wherein the antisense oligonucleotide has a 17-deoxynucleotide region flanked on its 5' and 3' ends with one or two 2'-O(2-methoxyethyl) nucleotides. In a further embodiment, the antisense oligonucleotide specifically hybridizes to and reduces expression of GCGR. In a further embodiment, at least one internucleoside linkage is a phosphorothioate linkage. In a further embodiment, at least one cytosine is a 5-methylcytosine.

In a particular embodiment, the antisense oligonucleotide has the nucleobase sequence of SEQ ID: 2, wherein the antisense oligonucdeotide has a 18-deoxynucleotide region flanked on its 5' and 3' ends with one 2'-O(2-methoxyethyl) nucleotides. In a further embodiment, the antisense oligonucleotide specifically hybridizes to and reduces expression of GCGR. In a further embodiment, at least one internucleoside linkage is a phosphorothioate linkage. In a further embodiment, at least one cytosine is a 5-methylcytosine.

Also contemplated herein is a pharmaceutical composition comprising an antisense oligonucleotide of the invention and optionally a pharmaceutically acceptable carrier, diluent, enhancer or excipient. The compounds of the invention can also be used in the manufacture of a medicament for the treatment of diseases and disorders related to glucagon effects mediated by GCGR.

Embodiments of the present disclosure include methods of reducing the expression of GCGR in tissues or cells comprising contacting said cells or tissues with an antisense oligonucleotide or pharmaceutical composition of the invention, methods of decreasing blood glucose levels, blood triglyceride levels, or blood cholesterol levels in an animal comprising administering to said animal an antisense oligonucleotide or a pharmaceutical composition of the invention. Blood levels may be plasma levels or serum levels. Also contemplated are methods of improving insulin sensitivity, methods of increasing GLP-1 levels and methods of inhibiting hepatic glucose output in an animal comprising administering to said animal an antisense oligonucleotide or a pharmaceutical composition of the invention. An improvement in insulin sensitivity may be indicated by a reduction in circulating insulin levels.

Other embodiments of the present disclosure include methods of treating an animal having a disease or condition associated with glucagon activity via GCGR comprising administering to said animal a therapeutically or prophylactically effective amount of an antisense oligonucleotide or a pharmaceutical composition of the invention. The disease or condition may be a metabolic disease or condition. In some embodiments, the metabolic disease or condition is diabetes, hyperglycemia, hyperlipidemia, metabolic syndrome X, obesity, primary hyperglucagonemia, insulin deficiency, or insulin resistance. In some embodiments, the diabetes is Type 2 diabetes. In some embodiments the obesity is diet-induced. In some embodiments, hyperlipidemia is associated with elevated blood lipid levls. Lipids include cholesterol and triglycerides. In one embodiment, the condition is liver steatosis. In some embodiments, the steatosis is steatohepatitis or non-alcoholic steatohepatitis.

Also disclosed are methods of preventing or delaying the onset of elevated blood glucose levels in an animal as well as methods of preserving beta-cell function in an animal using the oligomeric compounds delineated herein.

Compounds of the invention can be used to modulate the expression of GCGR in an animal in need thereof, such as a human. In one embodiment, the methods comprise the step of administering to said animal an effective amount of an antisense compound that reduces expression of GCGR RNA. In one embodiment, the antisense compounds of the present invention effectively reduce the levels or function of GCGR RNA. Because reduction in GCGR mRNA levels can lead to alteration in GCGR protein products of expression as well, such resultant alterations can also be measured. Antisense compounds of the present invention that effectively reduce the levels or function of GCGR RNA or protein products of expression is considered an active antisense compound. In one embodiment, the antisense compounds of the invention reduce the expression of GCGR causing a reduction of RNA by at least 10%, by at least 20%, by at least 25%, by at least 30%, by at least 40%, by at least 50%, by at least 60%, by at least 70%, by at least 75%, by at least 80%, by at least 85%, by at least 90%, by at least 95%; by at least 98%, by at least 99%, or by 100% as measured by an exemplified assay herein.

One having skill in the art armed with the antisense compounds illustrated herein will be able, without undue experimentation, to identify further antisense compounds.

### Antisense Mechanisms

"Antisense mechanisms" are all those involving hybridization of a compound with target nucleic acid, wherein the outcome or effect of the hybridization is either target degradation or target occupancy with concomitant stalling of the cellular machinery involving for example, transcription or splicing.

### Targets

As used herein, the terms "target nucleic acid" and "nucleic acid molecule encoding GCGR" have been used for convenience to encompass DNA encoding GCGR, RNA (including pre-mRNA and mRNA or portions thereof) transcribed from such DNA, and also cDNA derived from such RNA.

### Regions, Segments, and Sites

The targeting process usually also includes determination of at least one target region, segment, or site within the target nucleic acid for the antisense interaction to occur such that the desired effect, e.g., modulation of expression, will result. "Region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present invention, are defined as unique nucleobase positions within a target nucleic acid.

Once one or more target regions, segments or sites have been identified, oligomeric compounds are designed which are sufficiently complementary to the target, i.e., hybridize sufficiently well and with sufficient specificity, to give the desired effect.

### Variants

It is also known in the art that alternative RNA transcripts can be produced from the same genomic region of DNA. These alternative transcripts are generally known as "variants." More specifically, "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants." Consequently, mRNA variants are processed pre-mRNA variants and each unique pre-mRNA variant must always produce a unique mRNA variant as a result of splicing. These mRNA variants are also known as "alternative splice variants." If no splicing of the pre-mRNA variant occurs then the pre-mRNA variant is identical to the mRNA variant.

It is also known in the art that variants can be produced through the use of alternative signals to start or stop transcription and that pre-mRNAs and mRNAs can possess more that one start codon or stop codon. Variants that originate from a pre-mRNA or mRNA that use alternative start codons are known as "alternative start variants" of that pre-mRNA or RNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Consequently, the types of variants described herein are also suitable target nucleic acids.

### Modulation of Target Expression

"Modulation" means a perturbation of function, for example, either an increase (stimulation or induction) or a decrease (inhibition or reduction) in expression. As another example, modulation of expression can include perturbing splice site selection of pre-mRNA processing. "Expression" includes all the functions by which a gene's coded information is converted into structures present and operating in a cell. These structures include the products of transcription and translation. "Modulation of expression" means the perturbation of such functions. "Modulators" are those compounds that modulate the expression of GCGR and which comprise at least an 8-nucleobase portion which is complementary to a validated target segment.

Modulation of expression of a target nucleic acid can be achieved through alteration of any number of nucleic acid (DNA or RNA) functions. The functions of DNA to be modulated can include replication and transcription. Replication and transcription, for example, can be from an endogenous cellular template, a vector, a plasmid construct or otherwise. The functions of RNA to be modulated can include translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. One result of such interference with target nucleic acid function is modulation of the expression of GCGR. Thus, in one embodiment modulation of expression can mean increase or decrease in target RNA or protein levels. In another embodiment modulation of expression can mean an increase or decrease of one or more RNA splice products, or a change in the ratio of two or more splice products.

### Hybridization and Complementarity

"Hybridization" means the pairing of complementary strands of oligomeric compounds. While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances. An oligomeric compound is specifically hybridizable when there is a sufficient degree of complementarity to avoid non-specific binding of the oligomeric compound to non-target nucleic acid sequences under conditions in which specific binding is desired, i.e., under physiological conditions in the case of *in vivo* assays or therapeutic treatment, and under conditions in which assays are performed in the case of *in vitro* assays.

"Stringent hybridization conditions" or "stringent conditions" refer to conditions under which an oligomeric compound will hybridize to its target sequence, but to a minimal number of other sequences. Stringent conditions are sequence-dependent and will be different in different circumstances, and "stringent conditions" under which oligomeric compounds hybridize to a target sequence are determined by the nature and composition of the oligomeric compounds and the assays in which they are being investigated.

"Complementarity," as used herein, refers to the capacity for precise pairing between two nucleobases on one or two oligomeric compound strands. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be a complementary position. The oligomeric compound and the further DNA or RNA are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligomeric compound and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure, mismatch or hairpin structure). The oligomeric compounds of the present invention comprise at least 70%, or at least 75%, or at least 80%, or at least 85%, or at least 90%, or at least 92%, or at least 95%, or at least 97%, or at least 98%, or at least 99% sequence complementarity to a target region within the target nucleic acid sequence to which they are targeted. For example, an oligomeric compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an oligomeric compound which is 18 nucleobases in length having 4 (four) noncomplementary nucleobases which are flanked by two regions of complete complementarity with the target nucleic acid would have 77.8% overall complementarity with the target nucleic acid. Percent complementarity of an oligomeric compound with a region of a target nucleic acid can be determined routinely using BLAST programs (basic local alignment search tools) and PowerBLAST programs known in the art (Altschul et al., J. Mol. Biol., 1990,215,403-410; Zhang and Madden, Genome Res., 1997, 7,649-656). Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math.,1981, 2, 482-489).

### Oligomeric compounds

The term "oligomeric compound" refers to a polymeric structure capable of hybridizing to a region of a nucleic acid molecule. This term includes oligonucleotides, oligonucleosides, oligonucleotide analogs, oligonucleotide mimetics and chimeric combinations of these. Oligomeric compounds are routinely prepared linearly but can be joined or otherwise prepared to be circular. Moreover, branched structures are known in the art. An "antisense compound" or "antisense oligomeric compound" refers to an oligomeric compound that is at least partially complementary to the region of a nucleic acid molecule to which it hybridizes and which modulates (increases or decreases) its expression. Consequently, while all antisense compounds can be said to be oligomeric compounds, not all oligomeric compounds are antisense compounds. An "antisense oligonucleotide" is an antisense compound that is a nucleic acid-based oligomer. An antisense oligonucleotide can be chemically modified. Nonlimiting examples of oligomeric compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound.

"Chimeric" oligomeric compounds or "chimeras," in the context of this invention, are single-or double-stranded oligomeric compounds, such as oligonucleotides, which contain two or more chemically distinct regions, each comprising at least one monomer unit, i.e., a nucleotide in the case of an oligonucleotide compound.

A "gapmer" is defined as an oligomeric compound, generally an oligonucleotide, having a 2'-deoxyoligonucleotide region flanked by non-deoxyoligonucleotide segments. The central region is referred to as the "gap." The flanking segments are referred to as "wings." If one of the wings has zero non-deoxyoligonucleotide monomers, a "hemimer" is described.

### NAFLD

The term "nonalcoholic fatty liver disease" (NAFLD) encompasses a disease spectrum ranging from simple triglyceride accumulation in hepatocytes (hepatic steatosis) to hepatic steatosis with inflammation (steatohepatitis), fibrosis, and cirrhosis. Nonalcoholic steatohepatitis (NASH) occurs from progression of NAFLD beyond deposition of triglycerides. A second-hit capable of inducing necrosis, inflammation, and fibrosis is required for development ofNASH. Candidates for the second-hit can be grouped into broad categories: factors causing an increase in oxidative stress and factors promoting expression of proinflammatory cytokines. It has been suggested that increased liver triglycerides lead to increased oxidative stress in hepatocytes of animals and humans, indicating a potential cause-and-efeet relationship between hepatic triglyceride accumulation, oxidative stress, and the progression of hepatic steatosis to NASH (Browning and Horton, J. Clin. Invest., 2004, 114, 147-152). Hypertriglyceridemia and hyperfattyacidemia can cause triglyceride accumulation in peripheral tissues (Shimamura et al., Biochem. Biophys. Res. Commun. 2004, 322, 1080-1085). One embodiment of the present disclosure a method of reducing lipids in the liver of an animal by administering a prophylactically or therapeutically effective amount of an oligomeric compound of the invention. Another embodiment of the present invention is a method of treating hepatic steatosis in an animal by administering a prophylactically or therapeutically effective amount of an oligomeric compound of the invention. In some embodiments, the steatosis is steatohepatitis. In some embodiments, the steatotis is NASH.

### Chemical Modifications

### Modified and Alternate Nucleobases

The oligomeric compounds of the invention also include variants in which a different base is present at one or more of the nucleotide positions in the compound. For example, if the first nucleotide is an adenosine, variants may be produced which contain thymidine, guanosine or cytidine at this position. This may be done at any of the positions of the oligomeric compound. These compounds are then tested using the methods described herein to determine their ability to reduce expression of GCGR mRNA.

Oligomeric compounds can also include nucleobase (often referred to in the art as heterocyclic base or simply as "base") modifications or substitutions. As used herein, "unmodified" or "natural" nucleobases include the purine bases adenine (A) and guanine (G), and the pyrimidine bases thymine (T), cytosine (C) and uracil (U). A "substitution" is the replacement of an unmodified or natural base with another unmodified or natural base. "Modified" nucleobases mean other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g. 9-(2-aminoethoxy)-H-pyrimido(5,4-b)(1,4)benzoxazin-2(3H)-one), carbazole cytidine (2H-pyrimido(4,5-b)indol-2-one), pyridoindole cytidine (H-pyrido(3',2':4,5)pyrrolo(2,3-d)pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613, and those disclosed by Sanghvi, Y.S., Chapter 15, Antisense Research and Applications, pages 289-302, Crooke, S.T. and Lebleu, B. , ed., CRC Press, 1993. Certain of these nucleobases are known to those skilled in the art as suitable for increasing the binding affinity of the compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C and are presently suitable base substitutions, even more particularly when combined with 2'-O-methoxyethyl sugar modifications. It is understood in the art that modification of the base does not entail such chemical modifications as to produce substitutions in a nucleic acid sequence.

Representative United States patents that teach the preparation of certain of the above noted modified nucleobases as well as other modified nucleobases include, but are not limited to, the above noted U.S. 3,687,808, as well as U.S.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941; and 5,750,692.

Oligomeric compounds of the present invention can also include polycyclic heterocyclic compounds in place of one or more of the naturally-occurring heterocyclic base moieties. A number of tricyclic heterocyclic compounds have been previously reported. These compounds are routinely used in antisense applications to increase the binding properties of the modified strand to a target strand. The most studied modifications are targeted to guanosines hence they have been termed G-clamps or cytidine analogs. Representative cytosine analogs that make 3 hydrogen bonds with a guanosine in a second strand include 1,3-diazaphenoxazine-2-one (Kurchavov, et al., Nucleosides and Nucleotides, 1997, 16, 1837-1846), 1,3-diazaphenothiazine-2-one, (Lin, K.-Y.; Jones, R. J.; Matteucci, M. J. Am. Chem. Soc. 1995, 117, 3873-3874) and 6,7,8,9-tetrafluoro-1,3-diazaphenoxazine-2-one (Wang, J.; Lin, K.-Y., Matteucci, M. Tetrahedron Lett. 1998, 39, 8385-8388). Incorporated into oligonucleotides these base modifications were shown to hybridize with complementary guanine and the latter was also shown to hybridize with adenine and to enhance helical thermal stability by extended stacking interactions (also see U.S. Pre-Grant Publications 20030207804 and 20030175906).

Further helix-stabilizing properties have been observed when a cytosine analog/substitute has an aminoethoxy moiety attached to the rigid 1,3-diazaphenoxazine-2-one scaffold (Lin, K.-Y.; Matteucci, M. J. Am. Chem. Soc. 1998, 120, 8531-8532). Binding studies demonstrated that a single incorporation could enhance the binding affinity of a model oligonucleotide to its complementary target DNA or RNA with a ΔTₘ of up to 18°C relative to 5-methyl cytosine (dC5^{me}), which is a high affinity enhancement for a single modification. On the other hand, the gain in helical stability does not compromise the specificity of the oligonucleotides.

Further tricyclic heterocyclic compounds and methods of using them that are amenable to use in the present invention are disclosed in United States Patents 6,028,183, and 6,007,992.

The enhanced binding affinity of the phenoxazine derivatives together with their uncompromised sequence specificity makes them valuable nucleobase analogs for the development of more potent antisense-based drugs. In fact, promising data have been derived from *in vitro* experiments demonstrating that heptanucleotides containing phenoxazine substitutions are capable to activate RNase H, enhance cellular uptake and exhibit an increased antisense activity (Lin, K-Y; Matteucci, M. J. Am. Chem. Soc. 1998, 120, 8531-8532). The activity enhancement was even more pronounced in case of G-clamp, as a single substitution was shown to significantly improve the *in vitro* potency of a 20mer 2'-deoxyphosphorothioate oligonucleotides (Flanagan, W. M.; Wolf, J.J.; Olson, P.; Grant, D.; Lin, K.-Y.; Wagner, R. W.; Matteucci, M. Proc. Natl. Acad. Sci. USA, 1999, 96, 3513-3518).

Further modified polycyclic heterocyclic compounds useful as heterocyclic bases are disclosed in but not limited to, the above noted U.S. Patent 3,687,808, as well as U.S. Patents: 4,845,205; 5,130,302; 5,134,066; 5,175,273; 5,367,066; 5,432,272; 5,434,257; 5,457,187; 5,459,255; 5,484,908; 5,502,177; 5,525,711; 5,552,540; 5,587,469; 5,594,121, 5,596,091; 5,614,617; 5,645,985; 5,646,269; 5,750,692; 5,830,653; 5,763,588; 6,005,096; and 5,681,941, and U.S. Pre-Grant Publication 20030158403.

### Combinations

Compositions of the invention can contain two or more oligomeric compounds. In another related embodiment, compositions of the present invention can contain one or more antisense compounds, particularly oligonucleotides, targeted to a first nucleic acid and one or more additional antisense compounds targeted to a second nucleic acid target. Alternatively, compositions of the present invention can contain two or more antisense compounds targeted to different regions of the same nucleic acid target. Two or more combined compounds may be used together or sequentially.

### Combination therapy

The compounds of the invention may be used in combination therapies, wherein an additive effect is achieved by administering one or more compounds of the invention and one or more other suitable therapeutic/prophylactic compounds to treat a condition. Suitable therapeutic/prophylactic compound(s) include, but are not limited to, glucose-lowering agents, anti-obesity agents, and lipid lowering agents. Glucose lowering agents include, but are not limited to hormones, hormone mimetics, or incretin mimetics (e.g., insulin, including inhaled insulin, GLP-1 or GLP-1 analogs such as liraglutide, or exenatide), DPP(IV) inhibitors, a sulfonylurea (e.g., acetohexamide, chlorpropamide, tolbutamide, tolazamide, glimepiride, a glipizide, glyburide or a gliclazide), a biguanide (metformin), a meglitinide (e.g., nateglinide or repaglinide), a thiazolidinedione or other PPAR-gamma agonists (e.g., pioglitazone or rosiglitazone) an alpha-glucosidase inhibitor (e.g., acarbose or miglitol), or an antisense compound not targeted to GCGR. Also included are dual PPAR-agonists (e.g., muraglitazar, being developed by Bristol-Myers Squibb, or tesaglitazar, being developed by Astra-Zeneca). Also included are other diabetes treatments in development (e.g. LAF237, being developed by Novartis; MK-0431, being developed by Merck; or rimonabant, being developed by Sanofi-Aventis). Anti-obesity agents include, but are not limited to, appetite suppressants (e.g. phentermine or Meridia™), fat absorption inhibitors such as orlistat (e.g. Xenical™), and modified forms of ciliary neurotrophic factor which inhibit hunger signals that stimulate appetite. Lipid lowering agents include, but are not limited to, bile salt sequestering resins (e.g., cholestyramine, colestipol, and colesevelam hydrochloride), HMGCoA-reductase inhibitors (e.g., lovastatin, pravastatin, atorvastatin, simvastatin, and fluvastatin), nicotinic acid, fibric acid derivatives (e.g., clofibrate, gemfibrozil, fenofibrate, bezafibrate, and ciprofibrate), probucol, neomycin, dextrothyroxine, plant-stanol esters, cholesterol absorption inhibitors (e.g., ezetimibe), CETP inhibitors (e.g. torcetrapib, and JTT-705) MTP inhibitors (eg, implitapide), inhibitors of bile acid transporters (apical sodium-dependent bile acid transporters), regulators of hepatic CYP7a, ACAT inhibitors (e.g. Avasimibe), estrogen replacement therapeutics (e.g., tamoxigen), synthetic HDL (e.g. ETC-216), anti-inflammatories (e.g., glucocorticoids), or an antisense compound not targeted to GCGR. One or more of these drugs may be combined with one or more of the antisense inhibitors of GCGR to achieve an additive therapeutic effect.

### Oligomer Synthesis

Oligomerization of modified and unmodified nucleosides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713) and US Publication No. 2005-0014713, which is herein incorporated by reference.

Oligomeric compounds of the present invention can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

### Oligomer Purification and Analysis

Methods of oligonucleotide purification and analysis are known to those skilled in the art. Analysis methods include capillary electrophoresis (CE) and electrospray-mass spectroscopy. Such synthesis and analysis methods can be performed in multi-well plates.

### Non limiting disclosure

While certain compounds and compositions of the present invention have been described with specificity in accordance with certain embodiments, the examples herein serve only to illustrate the compounds of the invention and are not intended to limit the same.

### Example 1

### Assaying Modulation of Expression

Modulation of GCGR expression can be assayed in a variety of ways known in the art. GCGR mRNA levels can be quantitated by, e.g., Northern blot analysis, competitive polymerase chain reaction (PCR), or real-time PCR. RNA analysis can be performed on total cellular RNA or poly(A)+ mRNA by methods known in the art. Methods of RNA isolation are taught in, for example, Ansubel, F.M. et al., Current Protocols in Molecular Biology, Volume 1, pp. 4.1.1-4.2.9 and 4.5.1-4.5.3, John Wiley & Sons, Inc., 1993.

Northern blot analysis is routine in the art and is taught in, for example, Ausubel, F.M. et al., Current Protocols in Molecular Biology, Volume 1, pp. 4.2.1-4.7-9, John Wiley & Sons, Inc., 1996. Real-time quantitative (PCR) can be conveniently accomplished using the commercially available ABI PRISM™ 7700 Sequence Detection System, available from PE-Applied Biosystems, Foster City, CA and used according to manufacturer's instructions.

Levels of proteins encoded by GCGR can be quantitated in a variety of ways well known in the art, such as immunoprecipitation, Western blot analysis (immunoblotting), ELISA or fluorescence-activated cell sorting (PACS). Antibodies directed to a protein encoded by GCGR can be identified and obtained from a variety of sources, such as the MSRS catalog of antibodies (Aerie Corporation, Birmingham, MI), or can be prepared via conventional antibody generation methods. Methods for preparation of polyclonal antisera are taught in, for example, Ausubel, F.M. et al., Current Protocols in Molecular Biology, Volume 2, pp. 11.12.1-11.12.9, John Wiley & Sons, Inc, 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F.M. et al., Current Protocols in Molecular Biology, Volume 2, pp. 11.2.1-11.2.22, John Wiley & Sons, Inc., 1991.

The effect of oligomeric compounds of the present invention on target nucleic acid expression can be tested in any of a variety of cell types provided that the target nucleic acid is present at measurable levels. The effect of oligomeric compounds of the present invention on target nucleic acid expression can be routinely determined using, for example, PCR or Northern blot analysis. Cell lines are derived from both normal tissues and cell types and from cells associated with various disorders (e.g. hyperproliferative disorders). Cell lines derived from multiple tissues and species can be obtained from American Type Culture Collection (ATCC, Manassas, VA), the Japanese Cancer Research Resources Bank (Tokyo, Japan), or the Centre for Applied Microbiology and Research (Wiltshire, United Kingdom).

Primary cells, or those cells which are isolated from an animal and not subjected to continuous culture, can be prepared according to methods known in the art or obtained from various commercial suppliers. Additionally, primary cells include those obtained from donor human subjects in a clinical setting (i.e. blood donors, surgical patients).

### Cell types

The effect of oligomeric compounds on target nucleic acid expression was tested in HepG2 cells.

The human hepatoblastoma cell line HepG2 was obtained from the American Type Culture Collection (Manassas, VA). HepG2 cells were routinely cultured in Eagle's MEM supplemented with 10% fetal bovine serum, 1 mM non-essential amino acids, and 1 mM sodium pyruvate (Invitrogen Life Technologies, Carlsbad, CA). Cells were routinely passaged by trypsinization and dilution when they reached approximately 90% confluence. Multiwell culture plates are prepared for cell culture by coating with a 1:100 dilution of type 1 rat tail collagen (BD Biosciences, Bedford, MA) in phosphate-buffered saline. The collagen-containing plates were incubated at 37°C for approximately 1 hour, after which the collagen was removed and the wells were washed twice with phosphate-buffered saline. Cells were seeded into 96-well plates (Falcon-Primaria #353872, BD Biosciences, Bedford, MA) at a density of approximately 8,000 cells/well for use in oligomeric compound transfection experiments.

### Treatment with oligomeric compounds

When cells reached appropriate confluency, they were treated with oligonucleotide using a transfection method as described. Other suitable transfection reagents known in the art include, but are not limited to, LIPOFECTAMINE™, OLIGOFECTAMINE™, and FUGENE™. Other suitable transfection methods known in the art include, but are not limited to, electroporation.

### LIPOFECTIN™

When cells reach 65-75% confluency, they are treated with oligonucleotide. Oligonucleotide is mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture iss incubated at room temperature for approximately 0.5 hours. For cells grown in 96-well plates, wells are washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture is replaced with fresh culture medium. Cells are harvested 16-24 hours after oligonucleotide treatment.

### CYTOFECTIN™

When cells reach 65-75% confluency, they are treated with oligonucleotide. Oligonucleotide is mixed with CYTOFECTIN™ (Gene Therapy Systems, San Diego, CA) in OPTI-MEM™-1 reduced serum medium (Invitrogen Life Technologies, Carlsbad, CA) to achieve the desired concentration of oligonucleotide and a CYTOFECTIN™ concentration of 2 or 4 µg/mL per 100 nM oligonucleotide. This transfection mixture is incubated at room temperature for approximately 0.5 hours. For cells grown in 96-well plates, wells are washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture is replaced with fresh culture medium. Cells are harvested 16-24 hours after oligonucleotide treatment.

### Control oligonucleotides

Control oligonucleotides are used to determine the optimal oligomeric compound concentration for a particular cell line. Furthermore, when oligomeric compounds of the invention are tested in oligomeric compound screening experiments or phenotypic assays, control oligonucleotides are tested in parallel with compounds of the invention. In some embodiments, the control oligonucleotides are used as negative control oligonucleotides, i.e., as a means for measuring the absence of an effect on gene expression or phenotype. In alternative embodiments, control oligonucleotides are used as positive control oligonucleotides, i.e., as oligonucleotides known to affect gene expression or phenotype. Control oligonucleotides are shown in Table 2. "Target Name" indicates the gene to which the oligonucleotide is targeted. "Species of Target" indicates species in which the oligonucleotide is perfectly complementary to the target mRNA. "Motif" is indicative of chemically distinct regions comprising the oligonucleotide. Certain compounds in Table 2 are chimeric oligonucleotides, composed of a central "gap" region consisting of 2'-deoxynucleotides, which is flanked on both sides (5' and 3') by "wings". The wings are composed of 2'-O-(2-methoxyethyl) nucleotides, also known as 2'-MOE nucleotides. The "Motif" of each gapmer oligonucleotide is illustrated in Table 2 and indicates the number of nucleotides in each gap region and wing, for example, "5-10-5" indicates a gapmer having a 10-nucleotide gap region flanked by 5-nucleotide wings. ISIS 29848 is a mixture of randomized oligomeric compound; its sequence is shown in Table 2, where N can be A, T, C or G. The internucleoside (backbone) linkages are phosphorothioate throughout the oligonucleotides in Table 2. Unmodified cytosines are indicated by ""C" in the nucleotide sequence; all other cytosines are 5-methylcytosines.

**Table 2**

| **Control oligonucleotides for cell line testing, oligomeric compound screening and phenotypic assays** | | | | | |
|---|---|---|---|---|---|
| **ISIS #** | **Target Name** | **Species of Target** | **Sequence (5' to 3')** | **Motif** | **SEQ ID NO** |
| 113131 | CD86 | Human | CGTGTGTCTGTGCTAGTCCC | 5-10-5 | 8 |
| 289865 | forkhead box O1A (rhabdomyosarcoma) | Human | GGCAACGTGAACAGGTCCAA | 5-10-5 | 9 |
| 25237 | integrin beta 3 | Human | GCCCATTGCTGGACATGC | 4-10-4 | 10 |
| 196103 | integrin beta 3 | Human | AGCCCATTGCTGGACATGCA | 5-10-5 | 11 |
| 148715 | Jagged 2 | Human; Mouse; Rat | TTGTCCCAGTCCCAGGCCTC | 5-10-5 | 12 |
| 18076 | Jun N-Terminal Kinase -1 | Human | CTTTC^{u}CGTTGGA^{u}C^{u}CCCTGGG | 5-9-6 | 13 |
| 18078 | Jun N-Terminal Kinase-2 | Human | GTGCG^{u}CG^{u}CGAG^{u}C^{u}C^{u}CGAAATC | 5-9-6 | 14 |
| 183881 | kinesin-like 1 | Human | ATCCAAGTGCTACTGTAGTA | 5-10-5 | 15 |
| 29848 | None | none | NNNNNNNNNNNNNNNNNNNN | 5-10-5 | 16 |
| 226844 | Notch (Drosophila) homolog 1 | Human; Mouse | GCCCTCCATGCTGGCACAGG | 5-10-5 | 17 |
| 105990 | Peroxisome proliferator-activated receptor gamma | Human | AGCAAAAGATCAATCCGTTA | 5-10-5 | 18 |
| 336806 | Raf kinase C | Human | TACAGAAGGCTGGGCCTTGA | 5-10-5 | 19 |
| 15770 | Raf kinase C | Mouse; Murine sarcoma virus; Rat | ATGCATT^{u}CTG^{u}C^{u}C^{u}C^{u}C^{u}CAAGGA | 5-10-5 | 20 |

The concentration of oligonucleotide used varies from cell line to cell line. To determine the optimal oligonucleotide concentration for a particular cell line, the cells are treated with a positive control oligonucleotide at a range of concentrations. Positive controls are shown in Table 2. For example, for human and non-human primate cells, the positive control oligonucleotide may be selected from ISIS 336806, or ISIS 18078. For mouse or rat cells the positive control oligonucleotide may be, for example, ISIS 15770. The concentration of positive control oligonucleotide that results in 80% reduction of the target mRNA, for example, rat Raf kinase C for ISIS 15770, is then utilized as the screening concentration for new oligonucleotides in subsequent experiments for that cell line. If 80% reduction is not achieved, the lowest concentration of positive control oligonucleotide that results in 60% reduction of the target mRNA is then utilized as the oligonucleotide screening concentration in subsequent experiments for that cell line. If 60% reduction is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 µM to 40 µM when the antisense oligonucleotide is transfected by electroporation.

### Example 2

### Real-time Quantitative PCR Analysis of GCGR mRNA Levels

Quantitation of GCGR mRNA levels was accomplished by real-time quantitative PCR using the ABI PRISM™ 7600, 7700, or 7900 Sequence Detection System (PE-Applied Biosystems, Foster City, CA) according to manufacturer's instructions.

Gene target quantities obtained by RT, real-time PCR were normalized using either the expression level of GAPDH, a gene whose expression is constant, or by quantifying total RNA using RiboGreen^{™} (Molecular Probes, Inc. Eugene, OR). Total RNA was quantified using RiboGreen^{™} RNA quantification reagent (Molecular Probes, Inc. Eugene, OR). 170 µL of RiboGreen^{™} working reagent (RiboGreen^{™} reagent diluted 1:350 in 10mM Tris-HCl, 1 mM EDTA, pH 7.5) was pipetted into a 96-well plate containing 30 µL purified cellular RNA. The plate was read in a CytoFluor 4000 (PE Applied Biosystems) with excitation at 485nm and emission at 530nm.

GAPDH expression was quantified by RT, real-time PCR, either simultaneously with the quantification of the target or separately. For measurement simultaneous with measurement of target levels, primer-probe sets specific to the target gene being measured were evaluated for their ability to be "multiplexed" with a GAPDH amplification reaction prior to quantitative PCR analysis. Multiplexing refers to the detection of multiple DNA species, in this case the target and endogenous GAPDH control, in a single tube, which requires that the primer-probe set for GAPDH does not interfere with amplification of the target.

Probes and primers for use in real-time PCR were designed to hybridize to target-specific sequences. Methods of primer and probe design are known in the art. Design of primers and probes for use in real-time PCR can be carried out using commercially available software, for example Primer Express®, PE Applied Biosystems, Foster City, CA. The primers and probes and the target nucleic acid sequences to which they hybridize are presented in Table 4. The target-specific PCR probes have FAM covalently linked to the 5' end and TAMRA or MGB covalently linked to the 3' end, where FAM is the fluorescent dye and TAMRA or MGB is the quencher dye.

After isolation, the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Compounds of the invention can be evaluated for their effect on human target mRNA levels by quantitative real-time PCR as described herein, using a primer-probe set designed to hybridize to human GCGR. For example:
Forward primer: TGCGGTTCCCCGTCTTC (incorporated herein as SEQ ID NO: 21)
Reverse primer: CTTGTAGTCTGTGTGGTGCATCTG (incorporated herein as SEQ ID NO: 22)
And the PCR probe:
FAM-CATCTTCGTCCGCATCG-MGB (incorporated herein as SEQ ID NO: 23), where FAM is the fluorescent dye and MGB is a non-fluorescent quencher dye.

Compounds of the invention can be evaluated for their effect on rat target mRNA levels by quantitative real-time PCR as described in other examples herein, using a primer-probe set designed to hybridize to rat GCGR. For example:
Forward primer: CAGTGCCACCACAACCTAAGC (incorporated herein as SEQ ID NO: 24)
Reverse primer: AGTACTTGTCGAAAGTTCTGTTGCA (incorporated herein as SEQ ID NO: 25)
And the PCR probe:
FAM- TGCTGCCCCCACCTACTGAGCTG-TAMRA (incorporated herein as SEQ ID NO: 26), where FAM is the fluorescent dye and TAMRA is the quencher dye.

Compounds of the invention can be evaluated for their effect on monkey target mRNA levels by quantitative real-time PCR as described in other examples herein, using a primer-probe set designed to hybridize to monkey GCGR. For example:
Forward primer: ACTGCACCCGCAACGC (incorporated herein as SEQ ID NO: 27)
Reverse primer: CACGGAGCTGGCCTTCAG (incorporated herein as SEQ ID NO: 28)
And the PCR probe:
FAM- ATCCACGCGAACCTGTTTGTGTCCTT-TAMRA (incorporated herein as SEQ ID NO: 29), where FAM is the fluorescent dye and TAMRA is the quencher dye.

Another example of a primer-probe set designed to hybridize to monkey GCGR is: Forward primer: GAACCTTCGACAAGTATTCCTGCT (incorporated herein as SEQ ID NO: 30)
Reverse primer: GGGCAGGAGATGTTGGCC (incorporated herein as SEQ ID NO: 31)
And the PCR probe:
FAM- CCAGACACCCCCGCCAATAACA-TAMRA (incorporated herein as SEQ ID NO: 32), where FAM is the fluorescent dye and TAMRA is the quencher dye.

### Example 3 Design of "gap-widened" antisense oligonucleotides targeting human GCGR

A series of oligomeric compounds were designed to target human GCGR (Genbank accession number: NM_000160.1, incorporated herein as SEQ ID NO: 1), with varying sizes of the deoxynucleotide gap and 2'-MOE wings. Each of the oligonucleotides is 20 nucleobases in length and has the same nucleobase sequence (GCACTTTGTGGTGCCAAGGC, incorporated herein as SEQ ID NO: 2), and therefore targets the same segment of SEQ ID NO: 1 (nucleobases 532 to 551). The compounds are shown in Table 3. Plain text indicates a deoxynucleotide, and nucleotides designated with bold, underlined text are 2'-O-(2-methoxyethyl) nucleotides. Internucleoside linkages are phosphorothioate throughout, and all cytosines are 5-methylcytosines. Indicated in Table 3 is the "motif" of each compound, indicative of chemically distinct regions comprising the oligonucleotide.

**Table 3**

| **Antisense compounds targeting human GCGR** | | | |
|---|---|---|---|
| **ISIS Number** | **Chemistry** | **SEQ ID NO:** | **Motif** |
| 310457 | GCACTTTGTGGTGCCAAGGC | 2 | 5-10-5 gapmer |
| 325448 | GCACTTTGTGGTGCCAAGGC | 2 | 2-16-2 gapmer |
| 325568 | GCACTTTGTGGTGCCAAGGC | 2 | 3-14-3 gapmer |

The 5-10-5 gapmer, ISIS 310457, was tested for its ability to reduce target mRNA levels in vitro. HepG2 cells were treated with ISIS 310457 using methods as described herein. ISIS 310457 was analyzed for its effect on human glucagon receptor mRNA levels by quantitative real-time PCR and was found to reduce expression of GCGR by about 96%.

### Example 4 Design of "gap-widened" antisense oligonucleotides targeting rat GCGR

A series of oligomeric compounds were designed to target rat GCGR (Genbank accession number: M96674.1, incorporated herein as SEQ ID NO: 3) with varying sizes of the deoxynucleotide gap and 2'-MOE wings. Each of the oligonucleotides tested has the same nucleobase sequence (GCACTTTGTGGTACCAAGGT, incorporated herein as SEQ ID NO: 4) and therefore targets the same segment of SEQ ID NO: 3 (nucleobases 402 to 421). The segment targeted by the rat oligonucleotides corresponds to the segment of human GCGR targeted by ISIS 310457 (SEQ ID NO: 2). The compounds are shown in Table 4. Plain text indicates a deoxynucleotide, and nucleotides designated with bold, underlined text are 2'-O-(2-methoxyethyl) nucleotides. Internucleoside linkages are phosphorothioate throughout, and all cytosines are 5-methylcytosines. Indicated in Table 4 is the "motif' of each compound indicative of chemically distinct regions comprising the oligonucleotide.

**Table 4**

| **Antisense compounds targeting rat GCGR** | | | |
|---|---|---|---|
| **ISIS Number** | **Chemistry** | **SEQ ID NO:** | **Motif** |
| 356171 | GCACTTTGTGGTACCAAGGT | 4 | 5-10-5 gapmer |
| 357368 | GCACTTTGTGGTACCAAGGT | 4 | Uniform deoxy |
| 357369 | GCACTTTGTGGTACCAAGGT | 4 | 1-18-1 gapmer |
| 357370 | GCACTTTGTGGTACCAAGGT | 4 | 1-17-2 gapmer |
| 357371 | GCACTTTGTGGTACCAAGGT | 4 | 2-16-2 gapmer |
| 357372 | GCACTTTGTGGTACCAAGGT | 4 | 3-14-3 gapmer |
| 357373 | GCACTTTGTGGTACCAAGGT | 4 | 4-12-4 gapmer |

### Example 5 Effects of antisense oligonucleotides targeting GCGR-in vivo rat study

In accordance with the present disclosure the oligonucleotides designed to target rat GCGR were tested in vivo. Male Sprague Dawley rats, eight weeks of age, were injected with 50, 25, 12.5, or 6.25 mg/kg of ISIS 356171, ISIS 357368, ISIS 357369, ISIS 357370, ISIS 357371, ISIS 357372, or ISIS 357373 twice weekly for 3 weeks for a total of 6 doses. Saline-injected animals served as a control. Each of the oligonucleotides tested has the same nucleobase sequence (GCACTTTGTGGTACCAAGGT, incorporated herein as SQ ID NO: 4), and the chemistry and motif of each compound is described above.

After the treatment period, rats were sacrificed and target nucleic acid levels were evaluated in liver. RNA isolation and target mRNA expression level quantitation are performed as described by other examples herein using RIBOGREEN™. RNA from each treatment group was assayed alongside RNA from the group treated with ISIS 356171. Results are presented in Table 5a, 5b, 5c, 5d, 5e, and 5f as a percentage of saline-treated control levels.

**Table 5a**

| **Reduction of target levels in liver of rats treated with 2-16-2 antisense oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **12.5** | **6.25** |
| **ISIS 356171** | 5-10-5 | 7 | 20 | 26 | 36 |
| **ISIS 357371** | 2-16-2 | 11 | 22 | 35 | 39 |

**Table 5b**

| **Reduction of target levels in liver of rats treated with 3-14-3 antisense oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **125** | **6.25** |
| **ISIS 356171** | 5-10-5 | 10 | 24 | 28 | 50 |
| **ISIS 357372** | 3-14-3 | 12 | 23 | 37 | 56 |

**Table 5c**

| **Reduction of target levels in liver of rats treated with 4-12-4 antisense oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **12.5** | **6.25** |
| **ISIS 356171** | 5-10-5 | 10 | 25 | 36 | 47 |
| **ISIS 357373** | 4-12-4 | 13 | 22 | 48 | 47 |

**Table 5d**

| **Reduction of target levels in liver of rats treated with 1-17-2 antisense oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **12.5** | **6.25** |
| **ISIS 356171** | 5-10-5 | 8 | 24 | 32 | 43 |
| **ISIS 357370** | 1-17-2 | 20 | 41 | 62 | 68 |

**Table 5e**

| **Reduction of target levels in liver of rats treated with 1-18-1 antisense oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **12.5** | **6.25** |
| **ISIS 356171** | 5-10-5 | 9 | 27 | 34 | 46 |
| **ISIS 357369** | 1-18-1 | 33 | 35 | 58 | 70 |

**Table 5f**

| **Reduction of target levels in liver of rats treated with uniform deoxy oligonucleotides targeted to GCGR** | | | | | |
|---|---|---|---|---|---|
| **Treatment** | **Motif** | **% Control** | | | |
| | | **Dose of oligonucleotide (mg/kg)** | | | |
| | | **50** | **25** | **12.5** | **6.25** |
| **ISIS 356171** | 5-10-5 | 8 | 23 | 30 | 45 |
| **ISIS 357368** | Uniform deoxy | 31 | 43 | 77 | 73 |

As shown in Tables 5a, 5b, 5c, 5d, and 5e the gap-widened antisense oligonucleotides were effective at reducing GCGR levels in vivo in a dose-dependent manner. Thus, one embodiment of the present disclosure is a method of reducing expression of GCGR levels in an animal comprising administering an antisense oligonucleotide targeting GCGR. In one embodiment, the antisense oligonucleotide comprises a sixteen deoxynucleotide gap flanked on both the 5' and 3' end with two 2'-O-(2-methoxyethyl) nucleotides.

In addition, oligonucleotide concentration in kidney and liver were determined. Methods to determine oligonucleotide concentration in tissues are known in the art (Geary et al., Anal. Biochem., 1999, 274, 241-248). Shown in Table 6 are the total oligonucleotide concentration and the concentration of full length oligonucleotide (in µg/g) in the kidney or liver of animals treated with 25 mg/kg of the indicated oligonucleotide. Total oligonucleotide is the sum of all oligonucleotides metabolites detected in the tissue.

**Table 6**

| **Concentration of oligonucleotide in liver and kidney** | | | | | |
|---|---|---|---|---|---|
| **Treatment** | **Motif** | **Kidney Total oligo** | **Kidney Full-length** | **Liver Total oligo** | **Liver Full-length** |
| **ISIS 356171** | 5-10-5 gapmer | 1814 | 1510 | 621 | 571 |
| **ISIS 356368** | Uniform deoxy | 801 | 183 | 282 | 62 |
| **ISIS 356369** | 1-18-1 | 1237 | 475 | 309 | 171 |
| **ISIS 356370** | 1-17-2 | 1127 | 590 | 370 | 271 |
| **ISIS 356371** | 2-16-2 | 871 | 515 | 345 | 253 |
| **ISIS 356372** | 3-14-3 | 1149 | 774 | 497 | 417 |
| **ISIS 356373** | 4-12-4 | 902 | 687 | 377 | 326 |

As shown in Table 6, the concentrations of the gap-widened oligonucleotides in kidney were generally reduced with respect to those found for ISIS 356171 in these tissues. Taken with the target reduction data shown in Table 5 wherein potency was maintained with ISIS 356371, ISIS 356372, and ISIS 356373 with respect to ISIS 356171, these data suggest that gap-widened oligos, particularly ISIS 356371, ISIS 356372, and ISIS 356373 are, in essence, more affective than ISIS 356171 at reducing target levels in the liver.

### Example 6 Physiological effects of antisense oligonucleotides targeting GCGR-in vivo rat study

To assess the physiological effects of GCGR reduction with the antisense compounds of the invention, plasma glucose levels were monitored throughout the study for each treatment group described in the previous example. Glucose levels were measured using routine clinical methods (for example, the YSI glucose analyzer, YSI Scientific, Yellow Springs, OH) prior to the start of treatment ("Pre-bleed"), and during each week of the treatment period. Results are presented in Table 7 in mg/dL for each treatment group.

**Table 7**

| **Effect of antisense inhibition of GCGR on plasma glucose levels** | | | | | | |
|---|---|---|---|---|---|---|
| **Treatment** | **Motif** | **Dose** | **Pre-bleed** | **Week1** | **Week2** | **Week3** |
| Saline | n/a | n/a | 144 | 139 | 126 | 136 |
| ISIS 356171 | 5-10-5 | 50mg/kg | 125 | 131 | 115 | 110 |
| ISIS 356171 | | 25 mg/kg | 133 | 134 | 126 | 127 |
| ISIS 356171 | | 12.5 mg/kg | 143 | 139 | 128 | 133 |
| ISIS 356171 | | 6.25 mg/kg | 137 | 134 | 127 | 133 |
| ISIS 357368 | Uniform deoxy | 50 mg/kg | 139 | 135 | 123 | 128 |
| ISIS 357368 | | 25 mg/kg | 146 | 135 | 127 | 145 |
| ISIS 357368 | | 125 mg/kg | 136 | 133 | 125 | 132 |
| ISIS 357368 | | 6.25 mg/kg | 137 | 135 | 124 | 131 |
| ISIS 357369 | 1-18-1 | 50 mg/kg | 137 | 134 | 120 | 127 |
| ISIS 357369 | | 25 mg/kg | 147 | 136 | 126 | 125 |
| ISIS 357369 | | 12.5 mg/kg | 144 | 136 | 130 | 130 |
| ISIS 357369 | | 6.25 mg/kg | 138 | 131 | 130 | 133 |
| ISIS 357370 | 1-17-2 | 50 mg/kg | 145 | 132 | 130 | 120 |
| ISIS 357370 | | 25 mg/kg | 151 | 133 | 131 | 132 |
| ISIS 357370 | | 12.5 mg/kg | 140 | 139 | 132 | 132 |
| ISIS 337370 | | 6.25 mg/kg | 139 | 131 | 131 | 130 |
| ISIS 357371 | 2-16-2 | 50 mg/kg | 155 | 134 | 130 | 126 |
| ISIS 357371 | | 25 mg/kg | 142 | 133 | 125 | 122 |
| ISIS 357371 | | 125 mg/kg | 142 | 142 | 135 | 132 |
| ISIS 357371 | | 6.25 mg/kg | 146 | 138 | 133 | 132 |
| ISIS 357372 | 3-14-3 | 30 mg/kg | 155 | 134 | 132 | 127 |
| ISIS 357372 | | 25 mg/kg | 172 | 138 | 138 | 125 |
| ISIS 357372 | | 12.5 mg/kg | 151 | 140 | 135 | 130 |
| ISIS 357372 | | 6.25 mg/kg | 140 | 142 | 130 | 133 |
| ISIS 357373 | 4-12-4 | 50 mg/kg | 153 | 134 | 121 | 116 |
| ISIS 357373 | | 25 mg/kg | 143 | 135 | 129 | 118 |
| ISIS 357373 | | 12.5 mg/kg | 146 | 141 | 129 | 135 |
| ISIS 357373 | | 6.25 mg/kg | 141 | 137 | 137 | 140 |

As shown in Table 7, animals treated with the antisense compounds targeting GCGR showed trends toward reduced glucose over the course of the study. Therefore, another embodiment of the present disclosure is a method of lowering glucose levels in an animal comprising administering to said animal an antisense oligonucleotide which reduces the expression of GCGR levels. In preferred embodiments, the antisense oligonucleotide is a gap-widened oligonucleotide. In one embodiment, the antisense oligonucleotide comprises a sixteen deoxynucleotide gap flanked on both the 5' and 3' end with two 2'-O-(2-methoxyethyl) nucleotides. In some embodiments, the antisense oligonucleotide comprises a fourteen deoxynucleotide gap flanked on both the 5' and 3' end with three 2'-O-(2-methoxyethyl) nucleotides or a twelve deoxynucleotide gap flanked on both the 5' and 3' end with four 2'-O-(2-methoxyethyl) nucleotides.

To examine the effects of reduction of GCGR on other elements in the glucagon pathway, the animals treated with the antisense compounds were also assessed for glucagon levels and glucagon like peptide-1 (GLP-1) levels at the end of the treatment period. Plasma levels of glucagon and active GLP-1 were determined using commercially available kits, instruments, or services (for example, by radioimmunoassay, ELISA, and/or Luminex immunoassay, and/or Linco Research Inc. Bioanalytical Services, St. Louis, MO). Average glucagon levels (in ng/mL) and GLP-1 levels (pM) for each treatment group are shown in Table 8.

**Table 8**

| **Effects of antisense inhibition of GCGR on glucagon and GLP-1 levels** | | | | |
|---|---|---|---|---|
| **Treatment** | **Motif** | **Dose** | **Glucagon (ng/mL)** | **GLP-1 (PM)** |
| Saline | n/a | n/a | 19 | 6 |
| ISIS 356171 | 5-10-5 | 50 mg/kg | 1003 | 29 |
| ISIS 356171 | | 25 mg/kg | 59 | 27 |
| ISIS 356171 | | 12.5 mg/kg | 38 | 14 |
| ISIS 356171 | | 6.25 mg/kg | 27 | 16 |
| ISIS 357368 | Uniform deoxy | 50 mg/kg | 27 | 17 |
| ISIS 357368 | | 25 mg/kg | 25 | 13 |
| ISIS 357368 | | 12.5 mg/kg | 15 | 16 |
| ISIS 357368 | | 6.25 mg/kg | 19 | 8 |
| ISIS 357369 | 1-18-1 | 50 mg/kg | 73 | 20 |
| ISIS 357369 | | 25 mg/kg | 29 | 10 |
| ISIS 357369 | | 12.5 mg/kg | 83 | 13 |
| ISIS 357369 | | 6.25 mg/kg | 22 | 7 |
| ISIS 357370 | 1-17-2 | 50 mg/kg | 64 | 14 |
| ISIS 357370 | | 25 mg/kg | 37 | 20 |
| ISIS 357370 | | 12.5 mg/kg | 31 | 26 |
| ISIS 357370 | | 6.25 mg/kg | 23 | 28 |
| ISIS 357371 | 2-16-2 | 50 mg/kg | 468 | 7 |
| ISIS 357371 | | 25 mg/kg | 90 | 17 |
| ISIS 357371 | | 12.5 mg/kg | 27 | 7 |
| ISIS 357371 | | 6.25 mg/kg | 29 | 21 |
| ISIS 357372 | 3-14-3 | 50 mg/kg | 350 | 26 |
| ISIS 357372 | | 25 mg/kg | 61 | 18 |
| ISIS 357372 | | 12.5 mg/kg | 31 | 25 |
| ISIS 357372 | | 6.25 mg/kg | 26 | 14 |
| ISIS 357373 | 4-12-4 | 50 mg/kg | 342 | 22 |
| ISIS 357373 | | 25 mg/kg | 102 | 21 |
| ISIS 357373 | | 12.5 mg/kg | 61 | 7 |
| ISIS 357373 | | 6.25 mg/kg | 37 | 10 |

As shown in Table 8, antisense reduction of GCGR causes increases in circulating glucagon levels as well as in circulating GLP-1 levels. Although trends toward reductions in plasma glucose levels were noted as in Table 7, no hypoglycemia was observed. Therefore, another embodiment of the present disclosure is a method of increasing CLP-1 levels in an animal by administering an antisense oligonucleotide targeting GCGR. In one embodiments, the antisense oligonucleotide comprises a sixteen deoxynucleotide gap flanked on both the 5' and 3' end with two 2'-O-(2-methoxyethyl) nucleotides. In some Embodiments, the antisense oligonucleotide comprises a fourteen deoxynucleotide gap flanked on both the 5' and 3' end with three 2'-O-(2-methoxyethyl) nucleotides or a twelve deoxynucleotide gap flanked on both the 5' and 3' end with four 2'-O-(2-methoxyethyl) nucleotides. In preferred embodiments the antisense oligonucleotide is a gap-widened oligonucleotide. In preferred embodiments, the antisense oligonucleotide comprises ISIS 357371, ISIS 357372, or ISIS 357373.

### Example 7 Effects of antisense oligonucleotides targeting GCGR-in vivo study in cynomolgus monkeys

To evaluate alterations in tissue distribution, potency, or therapeutic index caused by modification of the antisense oligonucleotide motif in a primate, cynomolgus monkeys were injected with ISIS 310457 (5-10-5 motif) or ISIS 325568 (2-16-2 motif) at doses of 3, 10, or 20 mg/kg per week. These antisense compounds show 100% complementarity to the monkey GCGR target sequence. Animals injected with saline alone served as controls. The duration of the study was 7 weeks, and the animals were dosed three times during the first week, followed by once-weekly dosing for 6 weeks. Each treatment group was comprised of 5 animals. One group treated with 20 mg/kg of ISIS 310457 and one group treated with 20 mg/kg of ISIS 325568 recovered for three weeks after cessation of dosing prior to sacrifice ("20 mg/kg recovery"). Other treatment groups were sacrificed at the end of the study. Liver tissues were collected to assess target reduction.

RNA isolation and target mRNA expression level quantitation were performed as described by other examples herein using RIBOGREEN™. Results are presented in Table 9 as a percentage of saline-treated control levels.

**Table 9**

| **Reduction of target levels in liver of monkeys treated with antisense oligonucleotides targeted to GCGR** | | | | | |
|---|---|---|---|---|---|
| **Treatment** | **Motif** | **% Control** | | | |
| | | **Dose of oligonucleotide** | | | |
| | | **20 mg/kg, recovery** | **20 mg/kg** | **10 mg/kg** | **3 mg/kg** |
| ISIS 310457 | 5-10-5 | 27 | 34 | 43 | 71 |
| ISIS 325568 | 2-16-2 | 43 | 45 | 54 | 49 |

As shown in Table 9, treatment with ISIS 310457 and 325568 caused decreases in GCGR levels at all of the doses tested, and reduction in target levels was still observed in the 20 mg/kg recovery groups. ISIS 325568 caused greater reduction than ISIS 310457 at the 3 mg/kg dose. Thus, one embodiment of the present disclosure is a method of reducing expression of GCGR levels in an animal comprising administering an antisense oligonucleotide targeting GCGR. In preferred embodiments, the antisense oligonucleotide is a gap-widened oligonucleotide. In one embodiment, the antisense oligonucleotide comprises a sixteen deoxynucleotide gap flanked on both the 5' and 3' end with two 2'-O-(2-methoxythyl) nucleotides. In some embodiments, the antisense oligonucleotide comprises a fourteen deoxynucleotide gap flanked on both the 5' and 3' end with three 2'-O-(2-methoxyethyl) nucleotides or a twelve deoxynucleotide gap flanked on both the 5' and 3' end with four 2°-O-(2-methoxyethyl) nucleotides. In one embodiment, the antisense oligonucleotide comprises ISIS 325568.

In addition, oligonucleotide concentration in kidney and liver were determined. Methods to determine oligonucleotide concentration in tissues are known in the art (Geary et al., Anal Biochem, 1999, 274, 241-248). Shown in Table 10 are the total concentration and the concentration of full length oligonucleotide (in µg/g) in the kidney or liver of animals treated with the indicated oligonucleotide.

**Table 10**

| **Concentration of oligonucleotide in liver and kidney** | | | | | | |
|---|---|---|---|---|---|---|
| **Treatment** | **Motif** | **Dose** | **Total oligo** | **Kidney Full-length** | **Liver Total oligo** | **Liver Full-length** |
| ISIS 310457 | 5-10-5 | 3 mg/kg | 471 | 423 | 449 | 330 |
| | | 10 mg/kg | 1011 | 911 | 710 | 606 |
| | | 20 mg/kg | 1582 | 1422 | 981 | 867 |
| | | 20 mg/kg recovery | 449 | 347 | 648 | 498 |
| ISIS 325568 | 2-16-2 | 3 mg/kg | 356 | 298 | 309 | 228 |
| | | 10 mg/kg | 830 | 685 | 477 | 339 |
| | | 20 mg/kg | 1390 | 1101 | 739 | 544 |
| | | 20 mg/kg recovery | 264 | 161 | 344 | 205 |

As shown in Table 10, the kidney concentration of the 5-10-5 motif oligonucleotide ISIS 310457 is higher than that measured for the 2-16-2 motif oligonucleotide ISIS 325568 at all concentrations tested. Taken with the target reduction data in Table 9 for the 2-16-2 motif oligonucleotide, these data suggest that the gap-widened oligonucleotide is more potent than the corresponding 5-10-5 motif oligonucleotide, providing a more robust lowering of target mRNA levels in the liver without enhanced accumulation of oligonucleotide.

### Example 8 Physiological effects of antisense oligonucleotides targeting GCGR-in vivo study in cynomolgus monkeys

To examine the effects of reduction of GCGR on other elements in the glucagon pathway, the animals treated with the antisense compounds as described in Example 7 were also assessed for glucagon levels and glucagon like peptide-1 (GLP-1) levels during each week of treatment. The recovery groups were tested for an additional three weeks after cessation of dosing. Monkeys were anesthetized prior to blood collection to avoid artifacts due to stress. Plasma levels of glucagon and active GLP-1 were determined using commercially available kits, instruments, or services (for example, by radioimmunoassay, ELISA, and/or Luminex immunoassay, and/or Linco Research Inc. Bioanalytical Services, St. Louis, MO). Average glucagon levels (in ng/mL) and GLP-1 levels (pM) for each treatment group are shown in Table 11.

**Table 11**

| **Effects of antisense inhibition of GCGR on glucagon and GLP-1 levels in cynomolgus monkeys** | | | | | | | | | | |
|---|---|---|---|---|---|---|---|---|---|---|
| **Treatment group** | **Day of treatment** | | | | | | | | | |
| | **1 (Baseline)** | **8** | **15** | **22** | **29** | **36** | **43** | **50** | **57** | **64** |
| | **GLP-1** | | | | | | | | | |
| Saline | 9 | 11 | 13 | 8 | 11 | 7 | 16 | n/a | n/a | n/a |
| 310457, 3 mg/kg | 7 | 11 | 13 | 5 | 9 | 8 | 10 | n/a | n/a | n/a |
| 310457,10 mg/kg | 8 | 7 | 13 | 5 | 7 | 8 | 6 | n/a | n/a | n/a |
| 310457, 20 mg/kg | 9 | 10 | 15 | 8 | 13 | 11 | 13 | n/a | n/a | n/a |
| 310457,20 | 9 | 10 | 16 | 10 | 13 | 13 | 11 | 12 | 12 | 9 |
| mg/kg, recovery | | | | | | | | | | |
| 325568, 3 mg/kg | 5 | 9 | 8 | 5 | 7 | 16 | 7 | n/a | n/a | n/a |
| 325568,10 mg/kg | 6 | 13 | 7 | 6 | 8 | 11 | 9 | n/a | n/a | n/a |
| 325568,20 mg/kg | 6 | 11 | 7 | 9 | 8 | 10 | 7 | n/a | n/a | n/a |
| 325568,20 | | | | | | | | | | |
| mg/kg, recovery | 7 | 11 | 7 | 7 | 9 | 9 | 7 | 11 | 9 | 11 |

| | **Glucagon** | | | | | | | | | |
|---|---|---|---|---|---|---|---|---|---|---|
| Saline | 202 | 242 | 250 | 220 | 213 | 221 | 210 | n/a | n/a | n/a |
| 310457,3 mg/kg | 189 | 204 | 188 | 181 | 137 | 177 | 230 | n/a | n/a | n/a |
| 310437,10 mg/hg | 183 | 368 | 350 | 386 | 381 | 594 | 689 | n/a | n/a | n/a |
| 310457,20 mg/kg | 190 | 285 | 386 | 488 | 621 | 842 | 754 | n/a | n/a | n/a |
| 310457,20 | 189 | 422 | 507 | 519 | 991 | 1023 | 996 | 1715 | 1786 | 1488 |
| mg/kg, recovery | | | | | | | | | | |
| 325568,3 mg/kg | 253 | 198 | 230 | 261 | 294 | 329 | 330 | n/a | n/a | n/a |
| 325568,10 mg/kg | 203 | 297 | 315 | 360 | 376 | 490 | 426 | n/a | n/a | n/a |
| 325568, 20 mag/kg | 160 | 213 | 251 | 379 | 508 | 423 | 403 | n/a | n/a | n/a |
| 325568,20 | 222 | 373 | 370 | 434 | 537 | 500 | 526 | 1513 | 792 | 970 |
| mg/kg recovery | | | | | | | | | | |

Another embodiment of the present disclosure a method of increasing GLP-1 levels in an animal by administering an antisense oligonucleotide targeting GCGR. In preferred embodiments, the antisense oligonucleotide is a gap-widened oligonucleotide. In one embodiment, the antisense oligonucleotide comprises a 16 deoxynucleotide gap flanked on both the 5' and 3' end with two 2'-O-(2-metboxyethyl) nucleotides. In some embodiments, the antisense oligonucleotide comprises a 14 deoxynucleotide gap flanked on both the 5' and 3' end with three 2'-O-(2-methoxyethyl) nucleotides or a 12 deoxynucleotide gap flanked on both the 5' and 3' end with four 2'-O-(2-methoxyethyl) nucleotides. In preferred embodiments, the antisense oligonucleotide is ISIS 325568. In another embodiment, the antisense oligonucleotide comprises ISIS 325568.

## Claims

1. An antisense oligonucleotide 13 to 26 nucleobases in length targeted to a nucleic acid molecule encoding human GCGR, wherein the oligonucleotide comprises a first region, a second region, and a third region, wherein said first region comprises at least 11 deoxynucleotides, wherein said second and third regions comprise 1 to 4 2'-O-(2-methoxyethyl) nucleotides, said second and third regions flanking the first region on the 5' and 3' ends of said first region, wherein said oligonucleotide has at least 85% sequence complementarity to a target region within the nucleic acid molecule encoding human GCGR, and wherein the oligonucleotide specifically hybridizes to and reduces expression of human GCGR.

2. The antisense oligonucleotide of claim 1, wherein the oligonucleotide comprises a deoxynucleotide region 11, 12, 13, 14, 15, 16, 17, or 18 nucleobases in length.

3. The antisense oligonucleotide of claim 1 or claim 2, wherein the oligonucleotide is 20 nucleobases in length.

4. The antisense oligonucleotide of claim 3 **characterized by**:
(i) a 16-deoxynucleotide region flanked on its 5' and 3' ends with two 2'-O-(2-methoxyethyl) nucleotides;
(ii) an 18-deoxynucleotide region flanked on its 5' and 3' ends with one 2'-O-(2-methoxyethyl) nucleotides;
(iii) a 17-deoxynucleotide region flanked on its 5' and 3' ends with one or two 2'-O-(2-methoxyethyl) nucleotides;
(iv) a 12-deoxynucleotide region flanked on its 5' and 3' ends with four 2'-O-(2-methoxyethyl) nucleotides; or
(v) a 14-deoxynucleotide region flanked on its 5' and 3' ends with three 2'-O-(2-methoxyethyl) nucleotides.

5. The antisense oligonucleotide of any preceding claim, wherein:
(i) at least one internucleoside linkage is a phosphorothioate linkage;
(ii) all of the internucleotide linkages are phosphorothioate linkages;
(iii) at least one cytosine is a 5-methylcytosine; or
(iv) all of the cytosines are 5-methylcytosines.

6. The antisense oligonucleotide of claim 1, **characterized by** a 16-deoxynucleotide region flanked on its 5' and 3' ends with two 2'-O-(2-methoxyethyl) nucleotides wherein each internucleotide linkage is a phosphorothioate linkage and each cytosine is a 5-methylcytosine.

7. The antisense oligonucleotide of any preceding claim, wherein said oligonucleotide has at least 90%, or at least 92%, or at least 95%, or at least 97%, or at least 98%, or at least 99%, sequence complementarity to a target region within the nucleic acid molecule encoding human GCGR.

8. The antisense oligonucleotide of any preceding claim, wherein the oligonucleotide is targeted to a nucleic acid molecule of SEQ ID NO:1, SEQ ID NO:5, SEQ ID NO:6 or SEQ ID NO:7.

9. The antisense oligonucleotide of any preceding claim, wherein the oligonucleotide is targeted to a nucleic acid molecule of SEQ ID NO:1.

10. A pharmaceutical composition comprising the antisense oligonucleotide of any preceding claim and optionally a pharmaceutically acceptable carrier, diluent, enhancer or excipient.

11. The pharmaceutical composition of claim 10, for use in reducing the expression of GCGR in tissues or cells.

12. Use of the pharmaceutical composition of claim 10 in the manufacture of a medicament for reducing the expression of GCGR in tissues or cells.

13. The pharmaceutical composition of claim 10, for use in:
(i) decreasing blood glucose levels in an animal;
(ii) increasing GLP-1 levels in an animal;
(iii) improving insulin sensitivity in an animal;
(iv) decreasing blood triglycerides in an animal;
(v) decreasing blood cholesterol levels in an animal;
(vi) preventing or delaying the onset of elevated blood glucose levels in an animal; or
(vii) preserving beta-cell function in an animal.

14. Use of the pharmaceutical composition of claim 10 in the manufacture of a medicament for:
(i) decreasing blood glucose levels in an animal;
(ii) increasing GLP-1 levels in an animal;
(iii) improving insulin sensitivity in an animal;
(iv) decreasing blood triglycerides in an animal;
(v) decreasing blood cholesterol levels in an animal;
(vi) preventing or delaying the onset of elevated blood glucose levels in an animal; or
(vii) preserving beta-cell function in an animal.

15. The pharmaceutical composition of claim 10, for use in treating an animal having a disease or condition associated with glucagon activity via GCGR.

16. Use of the pharmaceutical composition of claim 10 in the manufacture of a medicament for treating an animal having a disease or condition associated with glucagon activity via GCGR.

17. The pharmaceutical composition of claim 15 or the use of claim 16 wherein:
(i) the disease or condition is a metabolic disease or condition;
(ii) the disease or condition is diabetes, hyperglycemia, obesity, primary hyperglucagonemia, insulin deficiency, or insulin resistance; or
(iii) the disease or condition is Type 2 diabetes.

## Patentansprüche

1. Antisense-Oligonukleotid mit einer Länge von 13 bis 26 Nukleobasen, gerichtet auf ein für menschlichen GCGR codierendes Nukleinsäuremolekül als Ziel, wobei das Oligonukleotid einen ersten Bereich, einen zweiten Bereich und einen dritten Bereich umfasst, wobei der erste Bereich wenigstens 11 Desoxynukleotide umfasst, wobei der zweite Bereich und der dritte Bereich 1 bis 4 2'-O-(2-Methoxyethyl)-Nukleotide umfassen, wobei der zweite und der dritte Bereich den ersten Bereich am 5'- bzw. 3'-Ende des ersten Bereichs flankieren, wobei das Oligonukleotid eine Sequenzkomplementarität von wenigstens 85% zu einem Zielbereich innerhalb des für menschlichen GCGR codierenden Nukleinsäuremoleküls aufweist und wobei das Oligonukleotid spezifisch an menschlichen GCGR hybridisiert und dessen Expression reduziert.

2. Antisense-Oligonukleotid nach Anspruch 1, wobei das Oligonukleotid einen Desoxynukleotidbereich mit einer Länge von 11, 12, 13, 14, 15, 16, 17 oder 18 Nukleobasen umfasst.

3. Antisense-Oligonukleotid nach Anspruch 1 oder Anspruch 2, wobei das Oligonukleotid eine Länge von 20 Nukleobasen aufweist.

4. Antisense-Oligonukleotid nach Anspruch 3, **gekennzeichnet durch**:
(i) einen an seinem 5'- und 3'-Ende mit zwei 2`-O-(2-Methoxyethyl)-Nukleotiden flankierten Bereich mit 16 Desoxynukleotiden;
(ii) einen an seinem 5'- und 3'-Ende mit einem 2`-O-(2-Methoxyethyl)-Nukleotid flankierten Bereich mit 18 Desoxynukleotiden;
(iii) einen an seinem 5'- und 3'-Ende mit einem bzw. zwei 2`-O-(2-Methoxyethyl)-Nukleotiden flankierten Bereich mit 17 Desoxynukleotiden;
(iv) einen an seinem 5'- und 3'-Ende mit vier 2`-O-(2-Methoxyethyl)-Nukleotiden flankierten Bereich mit 12 Desoxynukleotiden; oder
(v) einen an seinem 5'- und 3'-Ende mit drei 2`-O-(2-Methoxyethyl)-Nukleotiden flankierten Bereich mit 14 Desoxynukleotiden.

5. Antisense-Oligonukleotid nach einem vorhergehenden Anspruch, wobei:
(i) es sich bei wenigstens einer Internukleosidverknüpfung um eine Phosphorothioat-Verknüpfung handelt;
(ii) es sich bei allen Internukleosidverknüpfungen um Phosphorothioat-Verknüpfungen handelt;
(iii) es sich bei wenigstens einem Cytosin um ein 5-Methylcytosin handelt; oder
(iv) es sich bei allen Cytosinen um 5-Methylcytosine handelt.

6. Antisense-Oligonukleotid nach Anspruch 1, **gekennzeichnet durch** einen an seinem 5'- und 3'-Ende mit zwei 2`-O-(2-Methoxyethyl)-Nukleotiden flankierten Bereich mit 16 Desoxynukleotiden, wobei es sich bei der Internukleosidverknüpfung jeweils um eine Phosphorothioat-Verknüpfung und bei dem Cytosin jeweils um ein 5-Methylcytosin handelt.

7. Antisense-Oligonukleotid nach einem vorhergehenden Anspruch, wobei das Oligonukleotid eine Sequenzkomplementarität von wenigstens 90% oder wenigstens 92% oder wenigstens 95% oder wenigstens 97% oder wenigstens 98% oder wenigstens 99% zu einem Zielbereich innerhalb des für menschlichen GCGR codierenden Nukleinsäuremoleküls aufweist.

8. Antisense-Oligonukleotid nach einem vorhergehenden Anspruch, wobei das Oligonukleotid auf ein Nukleinsäuremolekül von SEQ ID NO:1, SEQ ID NO:5, SEQ ID NO:6 oder SEQ ID NO:7 als Ziel gerichtet ist.

9. Antisense-Oligonukleotid nach einem vorhergehenden Anspruch, wobei das Oligonukleotid auf ein Nukleinsäuremolekül von SEQ ID NO:1 als Ziel gerichtet ist.

10. Pharmazeutische Zusammensetzung, umfassend das Antisense-Oligonukleotid nach einem vorhergehenden Anspruch sowie gegebenenfalls ein pharmazeutisch unbedenkliches Träger-, Verdünnungs-, Verstärkungsoder Hilfsmittel.

11. Pharmazeutische Zusammensetzung nach Anspruch 10 zur Verwendung beim Reduzieren der GCGR-Expression in Geweben oder Zellen.

12. Verwendung der pharmazeutischen Zusammensetzung nach Anspruch 10 bei der Herstellung eines Arzneimittels zum Reduzieren der GCGR-Expression in Geweben oder Zellen.

13. Pharmazeutische Zusammensetzung nach Anspruch 10 zur Verwendung beim:
(i) Absenken der Blutglukosespiegel in einem Tier;
(ii) Erhöhen der GLP-1-Spiegel in einem Tier;
(iii) Verbessern der Insulinempfindlichkeit in einem Tier;
(iv) Absenken von Bluttriglyceriden in einem Tier;
(v) Absenken der Blutcholesterinspiegel in einem Tier;
(vi) Verhindern oder Verzögern des Auftretens erhöhter Blutglukosespiegel in einem Tier; oder
(vii) Erhalten der Beta-Zellfunktion in einem Tier.

14. Verwendung der pharmazeutischen Zusammensetzung nach Anspruch 10 bei der Herstellung eines Arzneimittels zum:
(i) Absenken der Blutglukosespiegel in einem Tier;
(ii) Erhöhen der GLP-1-Spiegel in einem Tier;
(iii) Verbessern der Insulinempfindlichkeit in einem Tier;
(iv) Absenken von Bluttriglyceriden in einem Tier;
(v) Absenken der Blutcholesterinspiegel in einem Tier;
(vi) Verhindern oder Verzögern des Auftretens erhöhter Blutglukosespiegel in einem Tier; oder
(vii) Erhalten der Beta-Zellfunktion in einem Tier.

15. Pharmazeutische Zusammensetzung nach Anspruch 10 zur Verwendung bei der Behandlung eines Tiers mit einer Krankheit bzw. einem Leiden in Zusammenhang mit Glucagon-Aktivität über GCGR.

16. Verwendung der pharmazeutischen Zusammensetzung nach Anspruch 10 bei der Herstellung eines Arzneimittels zur Behandlung eines Tiers mit einer Krankheit bzw. einem Leiden in Zusammenhang mit Glucagon-Aktivität über GCGR.

17. Pharmazeutische Zusammensetzung nach Anspruch 15 oder Verwendung nach Anspruch 16, wobei:
(i) es sich bei der Krankheit bzw. dem Leiden um eine Stoffwechselkrankheit bzw. ein Stoffwechselleiden handelt;
(ii) es sich bei der Krankheit bzw. dem Leiden um Diabetes, Hyperglykämie, Adipositas, primäre Hyperglucagonämie, Insulinmangel oder Insulinresistenz handelt; oder
(iii) es sich bei der Krankheit bzw. dem Leiden um Typ-2-Diabetes handelt.

## Revendications

1. Oligonucléotide antisens de 13 à 26 nucléobases de longueur ciblé vers une molécule d'acide nucléique codant pour GCGR humain, où l'oligonucléotide comprend une première région, une deuxième région, et une troisième région, où ladite première région comprend au moins 11 désoxynucléotides, où lesdites deuxième et troisième régions comprennent de 1 à 4 2'-O-(2-méthoxyéthyl)-nucléotides, lesdites deuxième et troisième régions flanquant la première région aux extrémités 5' et 3' de ladite première région, où ledit oligonucléotide a au moins 85 % de complémentarité de séquence avec une région cible dans la molécule d'acide nucléique codant pour GCGR humain, et où l'oligonucléotide s'hybride spécifiquement avec et réduit l'expression de GCGR humain.

2. Oligonucléotide antisens de la revendication 1, où l'oligonucléotide comprend une région désoxynucléotidique de 11, 12, 13, 14, 15, 16, 17, ou 18 nucléobases de longueur.

3. Oligonucléotide antisens de la revendication 1 ou la revendication 2, où l'oligonucléotide est de 20 nucléobases de longueur.

4. Oligonucléotide antisens de la revendication 3 **caractérisé par** :
(i) une région de 16 désoxynucléotides flanquée à ses extrémités 5' et 3' par deux 2' -O-(2-méthoxyéthyl)-nucléotides ;
(ii) une région de 18 désoxynucléotides flanquée à ses extrémités 5' et 3' par un 2'-O-(2-méthoxyéthyl)-nucléotide ;
(iii) une région de 17 désoxynucléotides flanquée à ses extrémités 5' et 3' par un ou deux 2'-O-(2-méthoxyéthyl)-nucléotides ;
(iv) une région de 12 désoxynucléotides flanquée à ses extrémités 5' et 3' par quatre 2'-O-(2-méthoxyéthyl)-nucléotides ; ou
(v) une région de 14 désoxynucléotides flanquée à ses extrémités 5' et 3' par trois 2'-O-(2-méthoxyéthyl)-nucléotides.

5. Oligonucléotide antisens de l'une quelconque des revendications précédentes, dans lequel :
(i) au moins un lieur internucléoside est une lieur phosphorothioate ;
(ii) tous les lieurs internucléosides sont des lieurs phosphorothioate ;
(iii) au moins une cytosine est une 5-méthylcytosine ; ou
(iv) toutes les cytosines sont des 5-méthylcytosines.

6. Oligonucléotide antisens de la revendication 1, **caractérisé par** une région de 16 désoxynucléotides flanquée à ses extrémités 5' et 3' par deux 2'-O-(2-méthoxyéthyl)-nucléotides dans lequel chaque lieur internucléoside est un lieur phosphorothioate et chaque cytosine est une 5-méthylcytosine.

7. Oligonucléotide antisens de l'une quelconque des revendications précédentes, où ledit oligonucléotide a au moins 90 %, ou au moins 92 %, ou au moins 95 %, ou au moins 97 %, ou au moins 98 %, ou au moins 99 %, de complémentarité de séquence avec une région cible dans la molécule d'acide nucléique codant pour GCGR humain.

8. Oligonucléotide antisens de l'une quelconque des revendications précédentes, où l'oligonucléotide est ciblé vers une molécule d'acide nucléique de SEQ ID NO: 1, SEQ ID NO: 5, SEQ ID NO: 6 ou SEQ ID NO: 7.

9. Oligonucléotide antisens de l'une quelconque des revendications précédentes, dans lequel l'oligonucléotide est ciblé vers une molécule d'acide nucléique de SEQ ID NO: 1.

10. Composition pharmaceutique comprenant l'oligonucléotide antisens de l'une quelconque des revendications précédentes et facultativement un véhicule, diluant, adjuvant ou excipient pharmaceutiquement acceptable.

11. Composition pharmaceutique de la revendication 10, pour utilisation dans la réduction de l'expression de GCGR dans des tissus ou des cellules.

12. Utilisation de la composition pharmaceutique de la revendication 10 dans la fabrication d'un médicament pour réduire l'expression de GCGR dans des tissus ou des cellules.

13. Composition pharmaceutique de la revendication 10, pour utilisation dans :
(i) la diminution des taux de glycémie chez un animal ;
(ii) l'augmentation des taux de GLP-1 chez un animal ;
(iii) l'amélioration de l'insulinosensibilité chez un animal ;
(iv) la diminution des triglycérides sanguins chez un animal ;
(v) la diminution des taux sanguins de cholestérol chez un animal ;
(vi) la prévention ou retard de l'apparition de taux de glycémie élevés chez un animal ; ou
(vii) la préservation de la fonction des cellules bêta chez un animal.

14. Utilisation de la composition pharmaceutique de la revendication 10 dans la fabrication d'un médicament pour :
(i) diminuer les taux de glycémie chez un animal ;
(ii) augmenter les taux de GLP-1 chez un animal ;
(iii) améliorer l'insulinosensibilité chez un animal ;
(iv) diminuer les triglycérides sanguins chez un animal ;
(v) diminuer les taux sanguins de cholestérol chez un animal ;
(vi) prévenir ou retarder l'apparition de taux de glycémie élevés chez un animal ; ou
(vii) préserver la fonction des cellules bêta chez un animal.

15. Composition pharmaceutique de la revendication 10, pour utilisation dans le traitement d'un animal ayant une maladie ou affection associée à l'activité du glucagon via GCGR.

16. Utilisation de la composition pharmaceutique de la revendication 10 dans la fabrication d'un médicament pour traiter un animal ayant une maladie ou affection associée à l'activité glucagon via GCGR.

17. Composition pharmaceutique de la revendication 15 ou utilisation de la revendication 16 où :
(i) la maladie ou affection est une maladie ou affection métabolique ;
(ii) la maladie ou affection est le diabète, l'hyperglycémie, l'obésité, l'hyperglucagonémie primaire, une déficience en insuline, ou une insulinorésistance ; ou
(iii) la maladie ou l'affection est le diabète de type 2.
